# FASCICULE DE BREVET EUROPEEN

(11) **EP 1 196 403 B1**
(45) Date de publication et mention de la délivrance du brevet: **09.11.2005**
(21) Numéro de dépôt: 00946022.1
(22) Date de dépôt: 27.06.2000
(51) Int. Cl.: C07D 295/06, C07D 295/02, C07D 295/08, A61K 31/55, A61P 25/00

(54) **N-ARALKYL AMINES CYCLIQUES ANTIPSYCHOTIQUES**
ANTIPSYCHOTISCHE N-ARALKYL CYCLISCHE AMINE
ANTIPSYCHOTIC CYCLIC N-ARALKYL AMINES

(30) Priorité: 02.07.1999 FR 9908532
(43) Date de publication de la demande: 17.04.2002
(73) Titulaire: Sanofi-Aventis, 75013 Paris (FR)
(72) Inventeur: BOIGEGRAIN, Robert, F-34820 Assas (FR); BOURRIE, Martine, F-34980 Saint-Gely du Fesc (FR); LAIR, Pierre, F-31120 Goyrans (FR); PAUL, Raymond, F-34980 Saint-Gely du Fesc (FR); PONCELET, Martine, F-34270 Valflaunes (FR); VERNIERES, Jean-Claude, F-31600 Muret (FR)
(74) Mandataire: Monain, Patrice
(86) Numéro de dépôt international: PCT/FR2000/001790
(87) Numéro de publication internationale: WO 2001/002380

(56) Documents cités:
- EP-A- 0 461 986
- WO-A-89/12443
- WO-A-95/30659
- WO-A-98/04251
- FR-A- 2 159 369
- FR-A- 2 249 659
- DATABASE CHEMABS [en ligne] CHEMICAL ABSTRACTS SERVICE, COLUMBUS, OHIO, US; BASARAB, GREGORY S. ET AL: "Design of sterol reductase inhibitors. Insights into the binding conformation of tertiary amine fungicides" retrieved from STN Database accession no. 118:2375 XP002153652 & ACS SYMP. SER. (1992), 504(SYNTH. CHEM. AGROCHEM. III), 414-27 ,
- DATABASE CHEMABS [en ligne] CHEMICAL ABSTRACTS SERVICE, COLUMBUS, OHIO, US; ZHANG, MING QIANG ET AL: "Novel stereoselective calcium channel ligands of the diphenylalkylamine-type" retrieved from STN Database accession no. 119:130911 XP002153653 & BIOORG. MED. CHEM. LETT. (1992), 2(10), 1283-8 ,
- DATABASE CHEMABS [en ligne] CHEMICAL ABSTRACTS SERVICE, COLUMBUS, OHIO, US; OMIYA, TOSHIMICHI: "N-Cinnamyl-N'-benzhydrylpiperazines" retrieved from STN Database accession no. 83:97373 XP002153654 & JP 50 004086 A (FUJIKAWA AND CO., LTD., JAPAN) 16 janvier 1975 (1975-01-16)
- ZHANG, M. Q. ET AL: "Calcium antagonism and structure-affinity relationships of terfenadine, a histamine H1 antagonist, and some related compounds" J. PHARM. PHARMACOL., vol. 45, no. 1, 1993, pages 63-66, XP000964893
- ZHANG M -Q ET AL: "OPTICALLY ACTIVE ANALOGUES OF EBASTINE: SYNTHESIS AND EFFECT OF CHIRALITY ON THEIR ANTIHISTAMINIC AND ANTIMUSCARINIC ACTIVITY" CHIRALITY,US,WILEY-LISS, NEW YORK, vol. 6, no. 8, 1994, pages 631-641, XP000613077 ISSN: 0899-0042
- REITZ, ALLEN B. ET AL: "Orally Active Benzamide Antipsychotic Agents with Affinity for Dopamine D2, Serotonin 5-HT1A, and Adrenergic.alpha.1 Receptors" J. MED. CHEM. , vol. 41, no. 12, 1998, pages 1997-2009, XP002153651

## Description

La présente invention concerne des dérivés du benzène comprenant une amine cyclique se liant spécifiquement aux récepteurs sigma, notamment à ceux du système nerveux central, un procédé pour la préparation de ces composés et leur utilisation dans des compositions pharmaceutiques et plus particulièrement en tant qu'antipsychotiques.

Les récepteurs sigma ont été mis en évidence à l'aide de plusieurs ligands. En premier lieu, on peut citer des composés opiacés, les 6,7-benzomorphans ou SKF-10,047, plus particulièrement le composé chiral (+) SKF-10,047 (W. R. Martin *et al*., J. Pharmacol. Exp.Ther. 1976, *197*, 517-532 ; B. R. Martin *et al*., J. Pharmacol. Exp. Ther. 1984, *231*, 539-544). Parmi ces composés, les plus utilisés sont la (+) N-allylnormétazocine ou (+) NANM et la (+) pentazocine. Un neuroleptique, l'halopéridol, est également un ligand des récepteurs sigma, ainsi que le (+) 3-(3-hydroxyphényl)-1-propylpipéridine ou (+) 3-PPP (B. L. Largent *et al*., Proc. Nat. Acad. Sci. USA 1984, 81, 4983-4987).

Le brevet US 4,709,094 décrit des dérivés de la guanidine très actifs comme ligands spécifiques des récepteurs sigma, plus particulièrement on peut citer la di-(*o*-tolyl) guanidine ou DTG. La distribution anatomique des récepteurs sigma dans le cerveau a été étudiée par autoradiographie, après marquage de ces récepteurs par la DTG selon E. Weber *et al*., Proc. Nat. Acad. Sci. USA 1986, 83, 8784-8788, ainsi que par les ligands (+) SKF-10,047 et (+) 3-PPP selon B. L. Largent *et al*., J. Pharmacol. Exp. Ther. USA 1986, 238, 739-748. L'étude par autoradiographie a permis d'identifier nettement les récepteurs sigma du cerveau et de les distinguer des autres récepteurs des opiacés, ainsi que ceux de la phencyclidine. Les récepteurs sigma sont particulièrement abondants dans le système nerveux central et concentrés dans le tronc cérébral, le système limbique et les régions impliquées dans la régulation des émotions. On trouve également des récepteurs sigma dans différents tissus périphériques. Ainsi, on distingue deux sortes de récepteurs sigma. Les ligands de type (+) SKF-10,047 se fixent sélectivement sur les récepteurs sigma-1 alors que d'autres ligands comme la DTG, l'halopéridol ou la (+) 3-PPP présentent une grande affinité à la fois pour les récepteurs sigma-1 et sigma-2.

Le brevet EP 461 986 décrit des composés de formule : 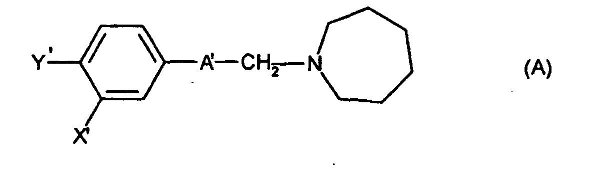 se liant sélectivement aux récepteurs sigma et possédant une activité antipsychotique.

Parmi cette série de composés, le chlorhydrate de 1-[3-(3-chloro-4-cyclohexylphényl)allyl]azépane, (Z) de formule : 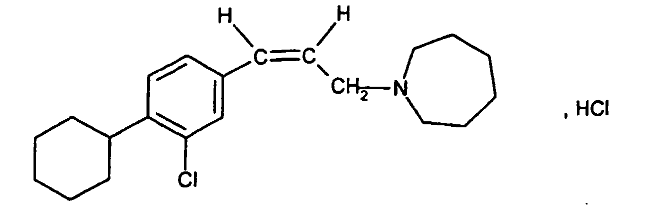 a particulièrement été étudié. On pourra se référer par exemple à Neuropharmacology 1993, *32* (6), 605-615 et Eur. J. Pharmacol. 1993, *231* (3), 465-467.

Les composés de formule (A) ont cependant une propriété spécifique qui pourrait être considérée comme un inconvénient. Il s'agit d'une propriété apparaissant lors de la métabolisation : la dépendance au cytochrome P450 nommé CYP 2D6.

En 1957, pour la première fois, on a envisagé que des différences héréditaires pouvaient être responsables des variations de réponse face aux médicaments.

Le métabolisme oxydatif montre des variations importantes selon les individus et les races. Les recherches effectuées ces 15 dernières années ont montrées que les variations de l'expression fonctionnelle de la famille du cytochrome P450 (CYP) multigénique est à l'origine de ces différences. Seulement quelques isoformes du cytochrome P450 parmi celles déjà caractérisées chez l'homme ont un rôle dans le métabolisme oxydatif des médicaments. On pourra se référer à Xenobiotica 1986, 16, 367-378. Jusqu'à présent les CYP 1A2, CYP 2A6, CYP 2C9, CYP 2D6, CYP 2C19, CYP 2E1 et CYP 3A4 ont été identifiés pour leur importance clinique. Actuellement, on estime que les CYP 3A4, CYP 2D6 et CYP 2C9 sont responsables à eux seuls (et à des degrés variables) de 90% du métabolisme d'oxydation des médicaments. Bien que l'expression fonctionnelle de ces isoformes soient régulée et influencée par un bon nombre de facteurs environnementaux, physiologiques, les facteurs génétiques ont l'influence la plus marquée, ce qui souligne le rôle important que joue le polymorphisme dans l'oxydation des médicaments. Un certain nombre de ces polymorphismes ont été étudiés (particulièrement ceux du CYP 2C19 et du CYP 2D6). Plus particulièrement, l'importance clinique du polymorphisme du CYP 2D6 dans la 4-hydroxylation de la debrisoquine a été démontrée (Clin. Pharmacol. Ther. 1991, 50, 233-238). Le polymorphisme génétique du CYP 2D6 est responsable du métabolisme problématique de plus de 30 médicaments importants et affecte jusqu'à 10% de la population caucasienne (métaboliseurs lents). II a été maintenant montré que cette isoforme contrôle la biotransformation de médicaments comme les anti-arythmiques, les β-bloquants, les anti-hypertenseurs, les antianginaux, les neuroleptiques et les antidépresseurs. A part quelques exceptions, ces médicaments sont utilisés en médecine psychiatrique et cardio-vasculaire pour le traitement à long terme.

Les conséquences pharmacocinétiques sont surtout d'ordre quantitatif : les sujets métaboliseurs lents ont un taux de produit inchangé plus élevé que les autres. Ces différences quantitatives ont un impact clinique considérable pour les molécules qui ont un index thérapeutique réduit.

La génétique influence donc fortement les différences d'efficacité et d'effets secondaires observés selon les individus. Ainsi, il est important de déterminer si le métabolisme d'un médicament peut-être modifié en cas de déficience génétique d'un enzyme.

II a été maintenant trouvé, selon la présente invention, de nouveaux dérivés du benzène affins pour les récepteurs sigma notamment ceux du système nerveux central, pourvus d'une activité antipsychotique mais ayant un taux de métabolisation bas et/ou une implication nulle ou très faible du CYP 2D6 dans le processus d'oxydation.

Ainsi selon l'un de ses aspects, la présente invention concerne les composés de formule (I) : 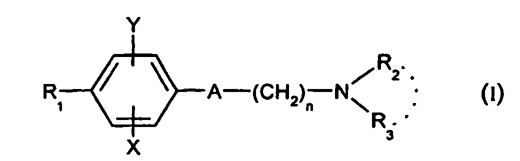 dans laquelle :
- A représente un groupe choisi parmi les suivants :
   -C≡C- ; -CH=CH- , -CH₂-CH₂-
- n est égal à 1 ou 2 ;
- X représente un atome d'hydrogène, de chlore ou de fluor ou un groupe méthyle ou méthoxy ;
- Y représente un atome d'hydrogène ou un atome de chlore ou de fluor ;
- R₁ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe (C₁-C₃)alcoxy ou trifluorométhyle ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ; ou R₁ représente un groupe phényle étant entendu que dans ce cas X ou Y est différent de l'hydrogène ; ou bien R₁ représente un groupe cyclohexyle étant entendu que dans ce cas X et Y sont différents de l'hydrogène ;
- R₂ et R₃ forment avec l'atome d'azote auquel ils sont liés une amine cyclique comprenant 5 à 8 chaînons ; un groupe morpholinyle éventuellement substitué en position 3 et 5 par un méthyle ; ou un groupe 4-phényl-1,2,3,6-tétrahydropyridinyle éventuellement substitué sur le phényle par un halogène ou un groupe trifluorométhyle, (C₁-C₄)alkyle ou (C₁-C₄)alcoxy ;
et les sels d'addition de ces composés avec des acides pharmaceutiquement acceptables, ainsi que leurs solvats et hydrates.

Par alkyle, on entend un radical monovalent hydrocarboné, saturé, linéaire ou ramifié.

Par (C₁-C₄)alkyle, on entend un radical alkyle comprenant de 1 à 4 atomes de carbone.

Par alcoxy, on entend un radical O-alkyle.

Parmi ces composés de formule (I), on préfère ceux dans lesquels :
- A représente un groupe choisi parmi les suivants :
   -C≡C- ; -CH=CH- ; -CH₂-CH₂-
- n est égal à 1;
- X représente un atome d'hydrogène, de chlore ou un groupe méthyle ;
- Y représente un atome d'hydrogène ou de chlore ;
- R₁ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ou trifluorométhyle ; un groupe *tert*-butyle ou adamantyle 1 ou 2 ; ou R₁ représente un groupe phényle étant entendu que dans ce cas X ou Y sont différents de l'hydrogène ; ou bien R₁ représente un groupe cyclohexyle étant entendu que dans ce cas X et Y sont différents de l'hydrogène ;
- R₂ et R₃ forment avec l'atome d'azote auquel ils sont liés une amine cyclique comprenant 6 à 8 chaînons ;
et les sels d'addition de ces composés avec des acides pharmaceutiquement acceptables ainsi que leurs solvats et hydrates.

Parmi ces derniers composés de formule (I), on préfère particulièrement les composés de formule : 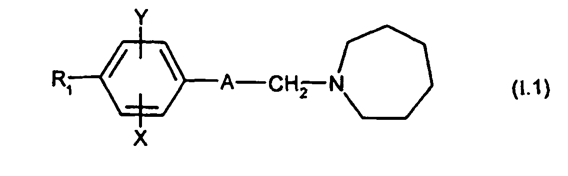 dans laquelle :
- A représente un groupe choisi parmi les suivants :
   -C≡C- ; -CH=CH- ; -CH₂-CH₂-
- X représente un atome d'hydrogène ou de chlore ;
- Y représente un atome d'hydrogène ou un atome de chlore ;
- R₁ représente un cyclohexyle monosubstitué, disubstitué, trisubstitué ou tétrasubstitué par un groupe méthyle ; un groupe phényle mono ou disubstitué par un atome de fluor, de chlore ou un groupe méthoxy ; un groupe *tert*-butyle ou adamantyle 1 ou 2 ; R₁ représente un groupe cyclohexyle ou phényle étant entendu que dans ce cas X et Y sont différents de l'hydrogène ;
et les sels d'addition de ces composés avec des acides pharmaceutiquement acceptables, ainsi que leurs solvats et hydrates.

Parmi ces derniers composés (I.1), on préfère les composés dans lesquels A représente le groupe -CH=CH- en particulier de configuration (Z).

Sont également préférés les composés de formule (I.1) dans lesquels X représente un atome de chlore de préférence en position 3 du phényle et Y représente un atome d'hydrogène et les sels d'addition de ces composés avec des acides pharmaceutiquement acceptables, ainsi que leurs solvats et hydrates.

Particulièrement, on préfère les composés de formule (I.1) dans lesquels R₁ représente un groupe phényle monosubstitué ou disubstitué par un atome de fluor ou de chlore ou un groupe méthoxy et les sels d'addition de ces composés avec des acides pharmaceutiquement acceptables, ainsi que leurs solvats et hydrates.

Particulièrement préférés sont les composés suivants :
- 1-[(Z)-3-(2-Chloro-3'-fluorobiphényl-4-yl)propèn-2-yl]azépane ;
- 1-[(Z)-3-(2-Chloro-3'-5'-difluorobiphényl-4-yl)propèn-2-yl]azépane ;
- et en particulier le 1-[(Z)-3-(2-Chloro-3'-méthoxybiphényl-4-yl)propèn-2-yl]azépane ;
ainsi que leurs sels avec des acides pharmaceutiquement acceptables, leurs solvats et hydrates.

Les sels des composés selon l'invention sont préparés selon des techniques bien connues de l'homme de l'art.

Les sels des composés de formule (I) selon la présente invention comprennent ceux avec des acides minéraux ou organiques qui permettent une séparation ou une cristallisation convenable des composés de formule (I), ainsi que des sels pharmaceutiquement acceptables. En tant qu'acide approprié, on peut citer : l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate. Parmi les sels des composés de formule (I), on préfère tout particulièrement les chlorhydrates.

Lorsqu'un composé selon l'invention présente un ou plusieurs carbones asymétriques, les isomères optiques de ce composé font partie intégrante de l'invention.

Lorsqu'un composé selon l'invention présente une stéréoisomérie par exemple de type axial-équatorial ou de type Z-E, l'invention comprend tous les stéréoisomères de ce composé.

La présente invention comprend les composés de formule (I) sous forme d'isomères purs mais également sous forme de mélange d'isomères en proportion quelconque. Les composés (I) sont isolés sous forme d'isomères purs par les techniques classiques de séparation : on pourra utiliser, par exemple des recristallisations fractionnées d'un sel du racémique avec un acide ou une base optiquement active dont le principe est bien connu ou les techniques classiques de chromatographies sur phase chirale ou non chirale, par exemple, on pourra utiliser la séparation sur gel de silice ou silice greffée C₁₈ en éluant avec des mélanges tels que solvants chloré/alcool. Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, alcools, phénolthiols ou des acides carboxyliques on entend les groupes protecteurs tels que ceux décrits dans Protective Groups in Organic Synthesis, Greene T.W. et Wuts P.G.M., ed. John Wiley et Sons, 1991 et dans Protecting Groups, Kocienski P.J., 1994, Georg Thieme Verlag.

L'homme de l'art sera à même de choisir les groupes protecteurs appropriés. Les composés de formule (I) peuvent comporter des groupes précurseurs d'autres fonctions qui sont générées ultérieurement en une ou plusieurs étapes.

La présente invention a également pour objet un procédé pour la préparation des composés de formule (I) caractérisé en ce que :
1) dans le cas où A représente le groupement -C≡C-:
   a) soit, si n = 1, on effectue une réaction de Mannich entre le dérivé phénylacétylénique de formule : 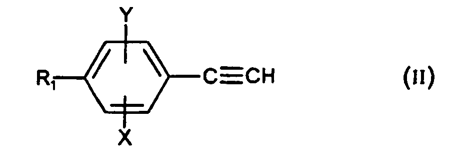 dans laquelle R₁, X et Y sont tels que définis pour (I), le formaldéhyde et l'amine (1) HNR₂R₃, R₂ et R₃ étant tels que définis pour (I) ;
   b) soit on effectue un couplage de Suzuki entre le composé de formule : 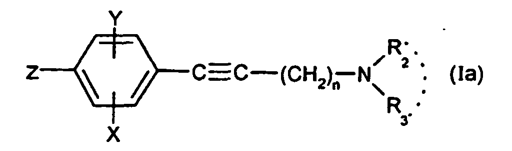 dans laquelle X, Y, n, R₂ et R₃ sont tels que définis pour (I) et Z représente un brome, un iode ou le groupe trifluorométhanesulfonate (OTf) et un dérivé boronique (2) de formule R₁-B(OR)₂ dans lequel R représente un atome d'hydrogène, un groupe alkyle ou aryle en présence d'une base et d'un catalyseur métallique ;
   c) soit lorsque R₁ représente un groupe cyclohexyle monosubstitué, disubstitué, trisubstitué ou tétrasubstitué par un groupe méthyle ; un groupe cycloheptyle, 4-tétrahydropyranyle, 4-tétrahydrothiopyranyle ou adamantyle, on effectue un couplage entre le composé (Ia) dans lequel Z représente un atome d'iode ou de brome avec la cétone (3) correspondant à R₁ représentée par 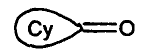 en présence d'une base pour obtenir intermédiairement le composé de formule : 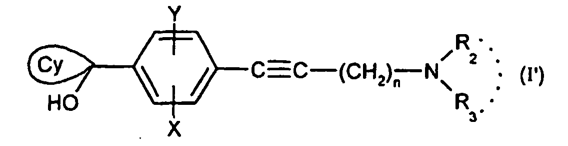 dans laquelle X, Y, n, R₂ et R₃ sont tels que définis pour (I) ; ledit composé (I') étant ensuite réduit dans des conditions sélectives ;
   d) soit on effectue une réaction de couplage entre l'amine de formule : 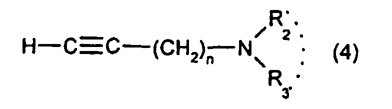
   dans laquelle n, R₂ et R₃ sont tels que définis pour (I) et le composé de formule : 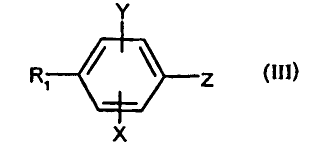 dans laquelle R₁, X et Y sont tels que définis pour (I) et Z représente un atome de brome, iode ou un groupe trifluorométhylsulfonate (triflate ou OTf) ;
2) dans le cas où A représente le groupe -CH=CH-, on effectue une hydrogénation par l'hydrogène naissant où en présence de cyclohexène du composé (I) dans lequel A représente le groupe acétylénique -C≡C- pour préparer le composé éthylénique (I) sous forme d'un mélange des isomères Z et E ou on effectue cette hydrogénation en présence d'un catalyseur métallique sur support pour préparer le composé éthylénique (I) sous forme Z, ou encore on fait réagir sur le composé (I) dans lequel A représente le groupe acétylénique -C≡C- un hydrure métallique pour préparer le composé éthylénique (I) sous forme E;
3) dans le cas où A représente le groupe -CH₂-CH₂-, on effectue une hydrogénation du composé (I) dans lequel A représente un groupe -CH=CH- ou -C≡C-.

A l'étape 1a du procédé selon l'invention, on procède à chaud, de préférence à une température comprise entre 80 et 90°C, dans un solvant polaire tel que le 1,2-diméthoxyéthane ou le 1,4-dioxane. Pour faciliter la réaction de condensation, on peut utiliser un catalyseur par exemple un sel métallique tel que le chlorure de cuivre II ou le chlorure de cuivre III.

A l'étape 1b du procédé, le couplage de Suzuki est de préférence effectué entre un composé (la) dans lequel Z représente OTf et le dérivé boronique (2) de formule R₁-B(OH)₂. La réaction est effectuée en présence d'une base, comme les hydroxydes, alcoxydes, phosphates ou carbonates alcalins ou alcalino terreux plus particulièrement le phosphate de potassium ou le carbonate de sodium. La réaction est effectuée en présence d'un catalyseur métallique, par exemple un catalyseur au cuivre, à l'étain ou de préférence au palladium tel que le tétrakis(triphénylphosphine)palladium éventuellement avec un halogénure du type chlorure de lithium jouant le rôle de cocatalyseur. On opère à chaud, à une température comprise entre 60 et 80°C dans un solvant inerte comme le toluène ou le 1,2-diméthoxyéthane ou de préférence en milieu biphasique toluène/solution aqueuse avec éventuellement une partie d'alcool comme l'éthanol.

Le couplage de Suzuki a été étudié dans de nombreuses publications comme par exemple Synth. Commun. 1981, *11* (7), 513-519 et J. Org. Chem. 1993, *58* (8), 2201-2208. Les acides boroniques (2) R₁-B(OH)₂ sont commerciaux ou synthétisés de façon classique à partir des dérivés halogénés correspondants de préférence bromés R₁Br par action par exemple du triméthylborate en présence d'une base comme le *tert*-butyllithium.

A l'étape 1c, le couplage est de préférence effectué sur un composé (Ia) dans lequel Z représente un atome de brome, en présence d'une base comme le *n*-butyllithium dans un solvant inerte de préférence le éther diéthylique à basse température, -80 à -70°C étant la gamme de température préférée. La réduction de (I') en (I) est effectuée dans des conditions sélectives par exemple selon la méthode décrite dans Tetrahedron, 1995, *51*, 11043-11062 par action du chlorotriméthylsilane et de l'iodure de sodium dans un mélange acétonitrile/solvant chloré comme le dichlorométhane suivie d'un traitement à l'acide acétique en présence de zinc ou encore par action d'acide iodhydrique ou par hydrogénation ionique par action de tétraborohydrure de sodium dans l'acide triflique.

A l'étape 1d du procédé, le couplage est effectué en présence d'un catalyseur au palladium, d'une ou plusieurs amines tertiaires et éventuellement de chlorure de lithium. On préférera utiliser un composé (III) dans lequel Z représente un triflate et on opérera en présence d'un catalyseur au palladium du type tétrakis(triphényl phosphine)palladium ou dichlorodi(triphénylphosphine)palladium et éventuellement d'un cocatalyseur comme l'iodure de cuivre. Dans le cas où Z représente un triflate, on utilisera également le chlorure de lithium. Ce couplage est de préférence effectué en présence de triéthylamine et de pyridine à reflux du mélange réactionnel. Pour ce type de couplage, appelé couplage de Sonogashira, on pourra se référer à J. Org. Chem. 1993, 58, 7368-7376 et 1998, 63, 1109-1118 ; Syn. Lett. 1995, 1115-1116 et Synthesis, 1987, 981.

Pour préparer les composés (I) dans lesquels A représente le groupe -CH=CH- sous forme Z, on effectue en général l'hydrogénation en présence de cyclohexène et d'un catalyseur métallique sur support, tel que le palladium sur sulfate de baryum ou carbonate de calcium ou le nickel de Raney ou de préférence le catalyseur de Lindlar, dans un solvant inerte pour la réaction, comme l'éther de pétrole ou un solvant alcoolique. Pour préparer les composés (I) sous forme E, on préfère utiliser comme hydrure métallique l'hydrure de diisobutylammonium (DIBALH) dans un solvant inerte comme le toluène.

Pour préparer les composés (I) dans lesquels A représente le groupe -CH₂-CH₂- l'hydrogénation est en général réalisée dans un alcool par exemple l'éthanol, en présence d'un catalyseur comme l'oxyde de platine ou de préférence le palladium sur charbon.

Pour les techniques de réduction des alcènes et alcynes utilisées ci-dessus, on pourra se référer à "Catalytic Hydrogenation. Techniques and Applications in Organic Chemistry", Robert L. Augustine, 1965, Marcel Dekker, Inc. New-York.

Le procédé général de préparation des composés (I) dans lesquels A représente le groupe acétylénique -C≡C- est décrit dans le SCHEMA 1 ci-après :

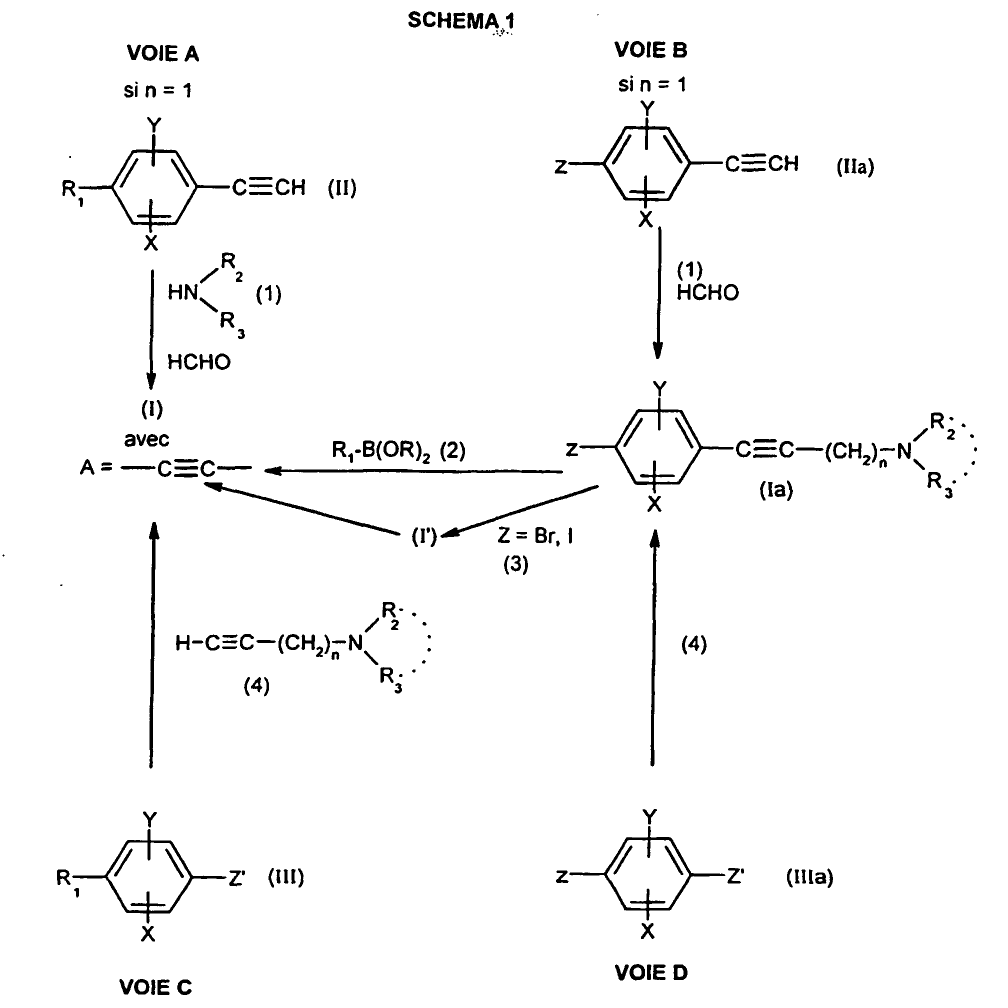

Dans le SCHÉMA 1, A = -C≡C- , et X, Y, n, R₁, R₂ et R₃ sont tels que définis pour (I), R représente un atome d'hydrogène, un groupe alkyle ou aryle, Z représente un atome de brome, d'iode ou un triflate et Z' représente un triflate lorsque Z représente un brome ou un iode sinon Z' représente un atome de brome ou d'iode. L'importance de la nature des substituants Z et Z' dans la réaction de couplage décrite VOIE D sera détaillée dans ce qui suit.

Le composé (II) est obtenu par traitement en milieu basique de la chloroacroléïne de formule : 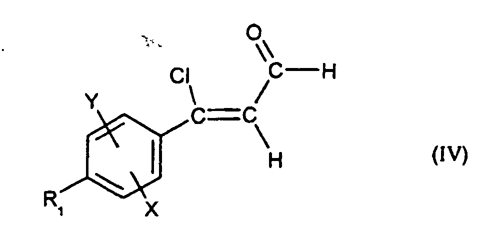 dans laquelle X, Y et R₁ sont tels que définis pour (I), de préférence par action de l'hydroxyde de sodium dans un solvant comme le tétrahydrofurane ou de préférence le 1,4-dioxane à la température de reflux du solvant.

La chloroacroléïne (IV) est préparée à partir de l'acétophénone de formule : 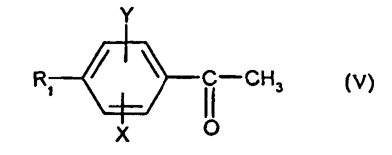 dans laquelle X, Y et R₁ sont tels que définis pour (I), par action d'un complexe de Vilsmeier. On utilise par exemple le chlorure de (chlorométhylène)diméthylammonium, réactif de Vilsmeier commercial, ou un réactif de Vilsmeier obtenu à partir d'une formamide disubstituée combinée avec le chlorure d'oxalyle, l'oxychlorure de phosphore ou le phosgène. On opère en général dans un solvant chloré ou un éther à une température comprise entre -20 et 40°C. Plus particulièrement, on utilise un réactif de Vilsmeier obtenu à partir du diméthylformamide et du chlorure d'oxalyle dans un solvant tel que le dichlorométhane ou le 1,2-diméthoxyéthane à des températures comprises entre -10 et 10°C.

Pour ce type de réaction, on pourra se référer par exemple à J. Chem. Soc. (C) 1970, 2484-2488 et Angew. Chem. Intemat. Ed. 1963, *2*, 98-99.

Les acétophénones (V) sont connues ou préparées selon des méthodes connues telles que celles décrites dans Gazz. Chim. Ital. 1949, *79*, 453-457 et J. Am. Chem. Soc. 1947, *69*, 1651-1652.

Le SCHEMA 2 illustre les méthodes utilisées pour la préparation des composés (V).

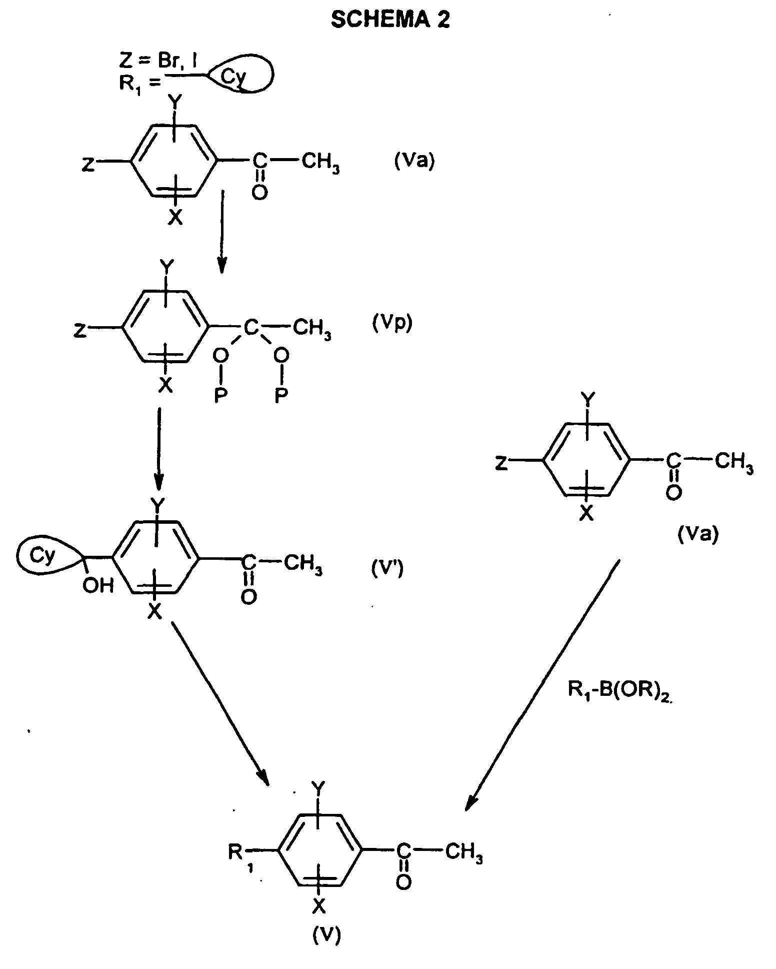

Dans le SCHEMA 2, X, Y et R₁ sont tels que définis pour (I), Cy est tel que défini précédemment pour (I'), Z représente un atome de brome, d'iode ou OTf, R représente un atome d'hydrogène, un groupe alkyle ou aryle et P représente un groupe protecteur de la fonction cétone comme un méthyle.

Les composés (V) peuvent être obtenus directement à partir des composés (Va) par action du composé boronique R₁-B(OR)₂ (2) comme décrit pour le passage de (Ia) à (I). On peut également protéger la fonction cétone du composé (Va) de manière classique par exemple par action d'orthoformate de trialkyle dans l'alcool correspondant en présence d'un acide comme l'acide *para*-toluènesulfonique. On obtient ainsi le composé (Vp) que l'on fait réagir sur la cétone 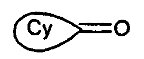 dans les conditions décrites pour le passage de (la) à (I'). On déprotège la fonction cétone par hydrolyse en milieu acide pour obtenir le composé (V'). Le dit composé (V') est alors réduit dans les conditions douces décrites pour le passage de (I') à (I).

Dans certains cas, par exemple, lorsque R₁ représente le groupe 4,4-diméthylcyclohexyle ou 4-tétrahydropyranyle, on pourra former intermédiairement le composé de formule : 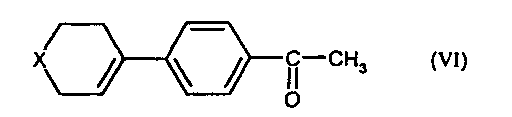 dans laquelle X = O ou -C(CH₃)₂ qui conduira, après protection préalable de la fonction cétone, hydrogénation par exemple en présence de palladium sur charbon dans le méthanol, suivie de la déprotection de la fonction cétone, au composé (V) souhaité.

Les composés (V) dans lesquels X et/ou Y est différent de l'hydrogène peuvent être obtenus à partir des composés (V) dans lesquels X = Y = H par des méthodes connues de l'homme de l'art. Par exemple, dans le cas ou X et/ou Y représente un atome de chlore, on effectue la chloration du noyau aromatique par action de chlore gazeux en présence d'un acide de Lewis de préférence le trichlorure d'aluminium, dans un solvant chloré comme le dichlorométhane, de préférence à 0°C.

Les composés (Va) sont commerciaux ou peuvent être préparés selon des méthodes connues de l'homme du métier.

Par exemple, dans le cas ou Z représente un triflate, le composé (Va) peut être préparé comme présenté dans le SCHEMA 3 :

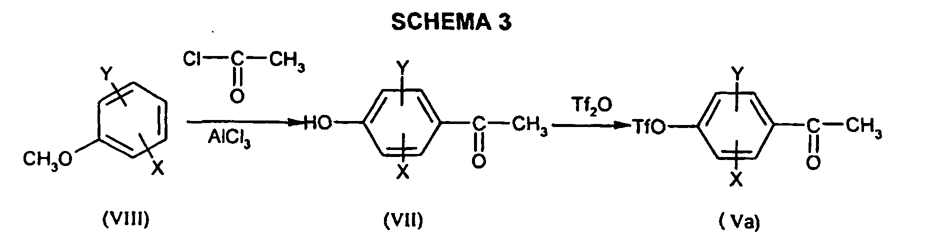

Dans le SCHEMA 3, X et Y sont tels que définis pour (I). Les composés (VIII) sont commerciaux ou préparés de manière classique.

Selon un autre de ses aspects, la présente invention a également pour objet les composés de formule (Ia) : 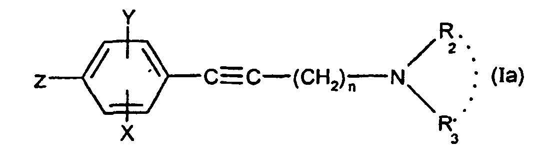 dans laquelle X, Y, n, R₂ et R₃ sont tels que définis pour (I) et Z représente un atome de brome, d'iode ou OTf. Ces composés sont nouveaux et constituent des intermédiaires clés dans la synthèse des composés (I).

La présente invention concerne également un procédé pour la préparation des dérivés (Ia) caractérisé en ce que :
- soit, lorsque n = 1, on effectue une réaction de Mannich entre le dérivé phénylacétylénique de formule : 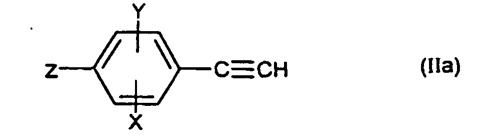
dans laquelle X et Y sont tels que définis pour (I) et Z représente un atome de brome , d'iode ou OTf, le formaldéhyde et l'amine (1) HNR₂R₃ ;
- soit on effectue une réaction de couplage entre l'amine de formule: 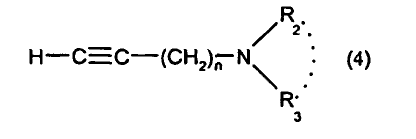
dans laquelle R₂, R₃ et n sont tels que définis pour (I) et le dérivé de formule : 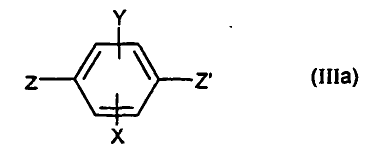 dans laquelle X et Y sont tels que définis pour (I), Z représente un atome de brome, d'iode ou un triflate et Z' représente un atome de brome ou d'iode si Z représente un triflate sinon Z' représente un triflate, en présence d'un catalyseur au palladium, d'une ou plusieurs amines tertiaires et éventuellement de chlorure de lithium.

La réaction de Mannich est effectuée dans les mêmes conditions que celles décrites pour le passage de (II) à (I).

Pour le couplage entre les composés (IIIa) et (4) on utilise une réaction de Sonogashira décrite pour le couplage des composés (III) et (4). Lorsque Z représente un triflate et Z' un atome de brome ou d'iode, on opère en absence de chlorure de lithium. Par contre, lorsque Z représente un atome de brome ou d'iode et Z' un triflate, on opère en présence de chlorure de lithium. L'utilisation de chlorure de lithium permet d'orienter la réaction de couplage.

Les propargylamines (4) (cas ou n = 1), sont préparées de manière classique par exemple selon Tetrah. Lett. 1989, *30* (13), 1679-1682 à partir de l'amine (1) HNR₂R₃ et du 3-bromopropyne par action du carbonate de potassium dans l'acétonitrile à une température comprise entre 50 et 80°C.

Les composés (III) dans lesquels Z = OTf sont obtenus classiquement à partir des alcools correspondants de formule : 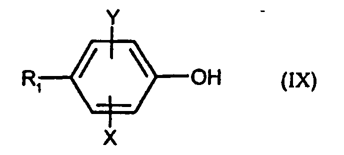 dans laquelle X, Y et R₁ sont tels que définis pour (I), par action de l'anhydride trifluorométhanesulfonique (anhydride triflique) dans la pyridine.

Les alcools (IX) sont eux-mêmes obtenus à partir des composés de formule : 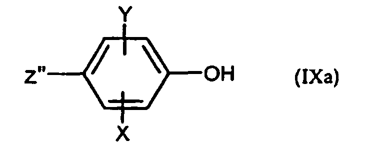 dans laquelle Z" représente un atome de brome ou d'iode selon les méthodes décrites précédemment pour le passage de (Ia) à (I) ou de (Va) à (V). Les composés (IXa) sont commerciaux ou préparés selon des techniques bien connues de l'homme de l'art.

Le composé (IIa) est préparé à partir de la chloroacroléine de formule : 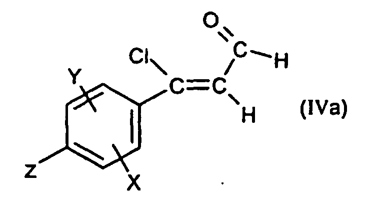 dans laquelle X, Y sont tels que définis pour (I) et Z représente un atome de brome, d'iode ou OTf, elle-même obtenue à partir de l'acétophénone de formule : 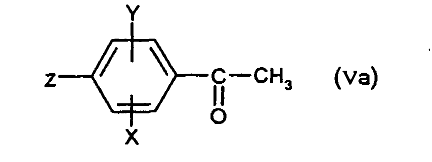 dans laquelle X, Y et Z sont tels que définis précédemment pour (IVa) selon les méthodes décrites pour le passage de (IV) à (II) et de (V) à (IV).

Les composés selon l'invention ont fait l'objet d'études biochimiques et pharmacologiques. Les composés de formule (I) ainsi que leurs sels, hydrates et solvats pharmaceutiquement acceptables se lient spécifiquement aux récepteurs sigma, notamment à ceux du système nerveux central.

L'affinité pour les récepteurs sigma-1 a été étudiée *in vitro* sur des membranes de cerveau de cobaye en utilisant la 3H-(+)-pentazocine comme ligand selon De Haven-Hudkins *et al*., Life Science 1993, 53, 41-48. La (+)-pentazocine se fixe spécifiquement sur les récepteurs sigma-1. On prépare selon les méthodes usuelles un fragment de membrane de cerveau de cobaye. La préparation membranaire (0,3 mg de protéine/ml) est incubée pendant 150 minutes à 37°C en présence de 0,5 nM de [³H]-(+)-pentazocine. La liaison non spécifique est déterminée en présence de 10 µM de (+)-pentazocine. Ensuite on filtre et rince 3 fois les membranes. On analyse la matière filtrée pour déterminer la fraction [³H]-pentazocine spécifiquement liée. Dans ces conditions, les composés de l'invention, dont des exemple suivent, ont des Cl₅₀ comprises entre 0,1 nM et 100 nM.

La capacité d'interaction des composés selon l'invention avec les récepteurs sigma-2 a été testée *in vitro* sur des membranes de rate de rat en utilisant comme ligand la [³H]-DTG selon R. Paul *et al*, Journal of Neuroimmunology 1994, *52*, 183-192. La préparation membranaire (1 ml) est incubée avec 2 nM de [³H]-DTG pendant 90 minutes à 20°C. La quantité de liaisons non spécifiques est estimée en présence de 10 µM de DTG ou d'halopéridol. Les membranes sont filtrées, lavées 2 fois et la matière filtrée est analysée pour déterminer la quantité de [³H]-DTG spécifiquement liée. Les composés selon l'invention présentent une activité sigma-2 comprise entre 1 nM et 500 nM.

L'activité sigma-1 a également été étudiée *in vivo* chez la souris avec le modèle des rotations induites par le ligand (+)-3PPP (0,05 µg/ml) selon P. Worms *et al*., Life Science 1986, *39,* 2199-2208. Les composés selon l'invention ont été administrés par voie intrapéritonéale à des doses de 0,25 mg/kg et par voie orale à des doses de 1 mg/kg.

L'activité antipsychotique potentielle des composés de l'invention a été étudiée comme suit selon différents tests décrits dans Neuropharmacology 1993, *32* (6), 605-615. Les composés selon l'invention ont été étudiés selon le modèle d'hyperactivité induite chez la souris par l'amphétamine (par voie intrapéritonéale à des doses de 2,5 mg/kg) et par la cocaïne (par voie intrapéritonéale à des doses de 16 mg/kg). Le test d'évitement actif chez le rat a également été utilisé. Ces tests ont montré l'activité anti-psychotique des composés selon l'invention dont les exemples figurent ci-après. Les composés de l'invention ont également fait l'objet d'études électrophysiologiques montrant qu'il existe une similitude entre les composés selon l'invention et les neuroleptiques classiques, aussi bien après administration unique qu'après administration répétée. Pour certains composés, les résultats obtenus mettent en évidence une grande sélectivité des produits selon l'invention en A10 (aire tegmentale ventrale = VTA) par rapport à A9 (substance noire) soit une augmentation du nombre des neurones dopaminergiques spontanément actifs uniquement en A10 et pas en A9. Cette propriété est très intéressante car la structure A9 est fortement impliquée dans les effets extrapyramidaux obtenus avec les antipsychotiques classiques (L.A. Chiodo et B.S. Bunney ; Catecholamines : Neuropharmacology and Central Nervous System - Theoritical aspects 1984, 369-391).

Les composés selon l'invention présentent, d'après les résultats observés lors de ces essais biochimiques et comportementaux une activité antipsychotique.

L'implication du CYP2D6 peut être mise en évidence par des études de métabolisme *in vitro* sur fractions microsomales hépatiques humaines. Le concept le plus utilisé est l'inhibition de l'enzyme par son inhibiteur spécifique : la quinidine utilisée à 20 fois son Kᵢ - Kᵢ étant la valeur absolue de la constante d'inhibition d'un principe actif vis-à-vis d'une enzyme.

Différents modèles permettent de mettre en évidence, dans une réaction métabolique spécifique, l'implication du CYP2D6.
- On peut utiliser des fractions microsomales hépatiques humaines qui contiennent l'ensemble des isoformes hépatiques humaines incubées en présence de co-facteur d'oxydoréduction (NADPH) et en absence ou en présence de quinidine à 20 fois son Kᵢ vis-à-vis du CYP2D6. La diminution de la métabolisation observée en présence de quinidine peut être associée à l'inhibition de l'isoforme CYP2D6, prouvant ainsi son éventuelle implication dans la ou les voies métaboliques étudiées.
- On peut également utiliser des fractions microsomales préparées à partir de cellules transfectées n'exprimant qu'une seule isoforme de cytochrome P-450 humaine (GENTEST Corp.).
- On peut également utiliser des hépatocytes humains en culture primaire qui sont capables d'effectuer des réactions métaboliques de phase I et II. Les incubations sont alors réalisées en cinétique sur 24 heures en absence et en présence de quinidine, inhibiteur puissant et spécifique du CYP2D6. On pourra se référer à J. Pharm. Exp. Ther. 1996, *277*, 321-332.

Les composés selon l'invention ont été particulièrement étudiés comme suit :
- le dit composé est incubé avec des fractions microsomales hépatiques humaines, du NADPH (co-facteur d'oxydo-réduction) ainsi qu'en présence ou en absence de quinidine. Le degré d'inhibition de la métabolisation observée en présence de quinidine reflète l'implication du CYP2D6 dans la métabolisation du dit composé. Cette approche est utilisable lorsque la métabolisation sur fractions microsomales hépatiques est d'une amplitude suffisante (c'est-à-dire supérieure ou égale à 10 % de la quantité de substrat de départ).
- Dans le cas où la métabolisation du dit composé sur microsomes hépatiques est trop faible pour pouvoir quantifier une inhibition avec précision, ou lorsque des vérifications supplémentaires sont nécessaires, des études complémentaires, plus approfondies, sur hépatocytes humains en culture primaire sont réalisées en cinétique sur 24 heures. Le degré d'implication du CYP2D6 dans la métabolisation hépatique globale est alors révélé par la diminution de la clairance intrinsèque du dit composé éventuellement observée en présence de quinidine.
- Les résultats obtenus montrent que les composés selon l'invention présentent un taux de métabolisation faible et/ou une faible implication du CYP2D6 dans le processus d'oxydation.

Aucun signe de toxicité n'est observé avec ces composés aux doses pharmacologiquement actives et leur toxicité est donc compatible avec leur utilisation comme médicaments.

Les composés de la présente invention sont donc particulièrement intéressants et pourront être utilisés avantageusement comme médicaments, notamment comme antipsychotiques, pour le traitement des troubles liés à l'ischémie cérébrale et les symptômes positifs et négatifs de la schizophrénie.

Les composés de l'invention sont également très intéressants pour leur activité neuroprotectrice, plus particulièrement en ce qui concerne l'apoptose. Les composés selon l'invention pourront également être utilisés en tant qu'analgésiques ou bien pour traiter les abus de drogues et les symptômes liés à la dépendance et ou sevrage des drogues.

Par ailleurs, les composés selon l'invention ont également une activité dans le domaine cardiovasculaire plus particulièrement pour la régulation des troubles du rythme cardiaque.

La présente invention a donc également pour objet des compositions pharmaceutiques contenant une dose efficace d'un composé selon l'invention ou d'un sel, d'un solvat ou d'un hydrate pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique, rectale ou intraocculaire, les principes actifs de formule (I) ci-dessus, ou leurs sels, solvats et hydrates éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet prophylactique ou thérapeutique désiré, la dose de principe actif peut varier entre 0,02 et 1 mg par kg de poids du corps et par jour.

Chaque dose unitaire peut contenir de 1 à 25 mg, de préférence de 5 à 12 mg, d'ingrédients actifs en combinaison avec un support pharmaceutique. Cette dose unitaire peut être administrée 1 à 5 fois par jour de façon à administrer un dosage journalier de 1 à 100 mg, de préférence de 5 à 60 mg.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec des matrices telles qu'un polymère ou une cyclodextrine (patch, formes à libération prolongée).

Les compositions de la présente invention peuvent contenir, à côté des produits de formule (I) ci-dessus ou de leurs sels, solvats et hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Les PREPARATIONS et EXEMPLES suivants illustrent l'invention sans toutefois la limiter.

Les points de fusion ont été mesurés selon la méthode de Micro-Kofler.

Les spectres de résonance magnétique nucléaire ont été effectués dans le diméthylsulfoxyde sauf mention contraire, à 200 MHz et les déplacements chimiques sont exprimés en p.p.m. Les abréviations utilisées ci-après sont les suivantes :
s = singulet ; m = multiplet ; d = doublet ; t = triplet ; q = quadruplet.

De manière conventionnelle, on numerotera par la suite le groupe phényle des composés (I) de la façon suivante :

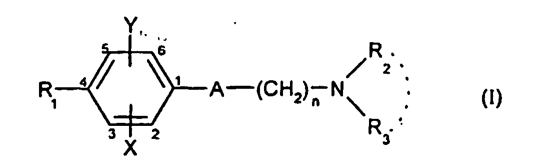

Dans les PREPARATIONS et EXEMPLES ci-après, n est égal à 1.

### PREPARATION 1

### 1-Bromo-4-(1,1-diméthoxyéthyl)benzène, composé V p

### (Vp): X = Y = H ; Z = Br ; P = CH₃

On agite pendant 6 heures à température ambiante un mélange de 19,905 g de 1-(4-bromophényl)éthanone, 101,4 ml de méthanol, 0,22 g d'acide *para*-toluènesulfonique hydraté et 19,9 ml de triméthylorthoformate. On neutralise la solution avec une solution d'hydroxyde de potassium à 1% dans le méthanol et concentre sous pression réduite. On reprend l'huile obtenue avec de l'éther de pétrole, élimine le précipité par filtration et évapore le filtrat sous pression réduite. On purifie le *composé Vp* par distillation ; rdt = 96% ; T_{Eb} = 82°C (sous une pression de 0,003 mbar).

### PREPARATION 2

### 1-[4-(1-Hydroxy-3,3,5,5-tétraméthylcyclohexyl)phényl]éthanone, composé V'.1

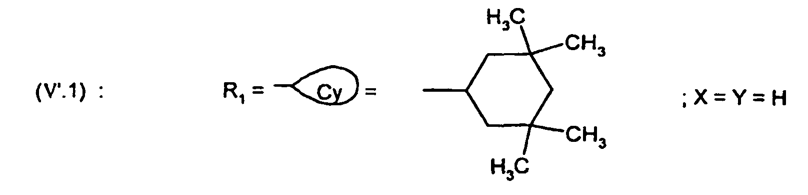

A -78°C, à une solution de 10 g de 1-bromo-4-(1,1-diméthoxyéthyl)benzène (*composé Vp*) dans 100 ml de tétrahydrofurane, on ajoute goutte à goutte 27,5 ml d'une solution à 1,6 M dans l'hexane de *n*-butyllithium. On agite le mélange réactionnel pendant 2 heures à cette température. On ajoute en 20 minutes une solution de 6,92 ml de 3,3,5,5-tétraméthylcyclohexanone dans 20 ml de tétrahydrofurane et agite le mélange réactionnel à -78°C pendant 1 heure. Après retour à température ambiante on additionne 140 ml d'une solution aqueuse saturée en chlorure d'ammonium. On décante, extrait la phase aqueuse au éther diéthylique, on réunit les phases organiques, les sèche sur sulfate de magnésium et évapore les solvants sous pression réduite. On purifie l'huile obtenue par chromatographie sur une colonne de gel de silice en éluant avec un mélange cyclohexane / acétate d'éthyle, 95 / 5 (v/v) ; rdt = 88% ; F = 135°C.

De la même manière, on prépare les composés suivants :

### [4-(Hydroxy-3,3-diméthylcyclohexyl)phényl]éthanone, composé V'.2

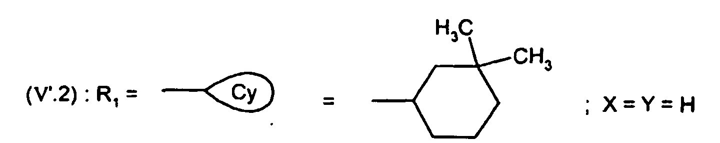

F = 99°C.

### 1-[4-(Hydroxyadamantan-2-yl)phényl]éthanone, composé V'.3

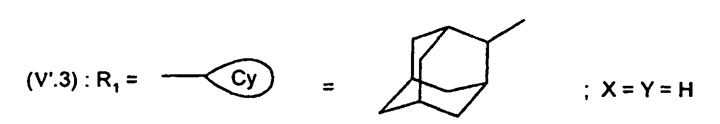

¹H RMN : 7,9 (d,2H) ; 7,6 (d,2H) ; 4,8 (s,1H) ; 2,6-1,4 (m,18H).

### PREPARATION 3

### 1-[4-(3,3,5,5-Tétraméthylcyclohexyl)phényl]éthanone, composé V.1

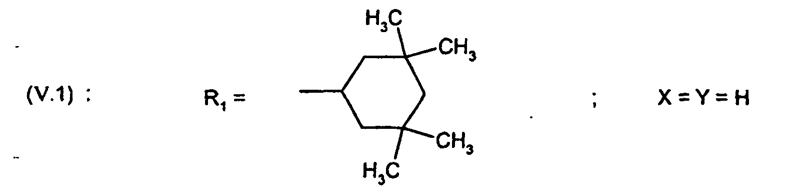

A une solution de 40,45 g de 1-[4-(hydroxy-3,3,5,5-tétraméthylcyclohexyl)phényl] éthanone (*composé V'.1*) et de 56,21 g d'iodure de sodium dans 230 ml d'acétonitrile anhydre, on additionne 38,1 ml de chlorotriméthylsilane en 45 minutes. Pendant l'addition, on maintient la température entre 35 et 40°C. Après 2 heures d'agitation, on additionne 40 ml d'acétonitrile et 39,4 ml d'acide acétique. Ensuite, on ajoute par portions 29,4 g de zinc en poudre fine, sous agitation et à température ambiante. On chauffe le mélange réactionnel à reflux sous forte agitation pendant 4 heures. Après retour à température ambiante, on filtre sur célite puis lave avec une solution aqueuse saturée en bicarbonate de sodium. On concentre la phase organique sous pression réduite et purifie l'huile obtenue par chromatographie sur une colonne de gel de silice en éluant avec un mélange cyclohexane / acétate d'éthyle , 95/5 (v/v) ; rdt = 68% ; F = 54°C.

De la même manière, on prépare les composés suivants :

### 1-[4-(3,3-Diméthylcyclohexyl)phényl]éthanone, composé V.2

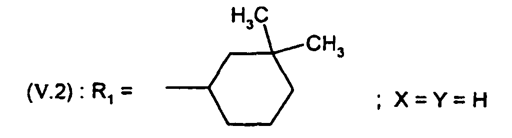

¹H RMN : 7,8 (d,2H) ; 7,2 (d,2H) ; 2,7 (m,1H) ; 2,5 (s,3H) ; 1,8-1,1 (m,8H) ; 1.0 (s,3H) ; 0,9 (s,3H).

### 1-(4-Adamantan-2-ylphényl)éthanone, composé V.3

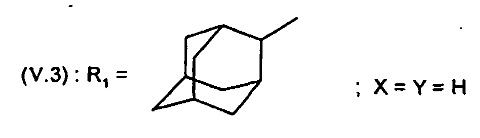

F = 75°C.

### PREPARATION 4

### 1-[3-Chloro-4-(3,3,5,5-tétraméthylcyclohexyl)phényl]éthanone, composé V.4

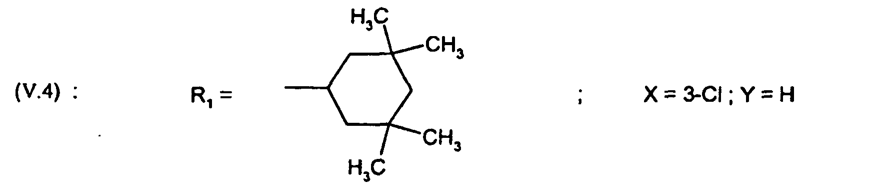

Sous atmosphère inerte, à 350 ml de dichlorométhane on additionne à 0°C 40,25 g de chlorure d'aluminium puis 5 g de 1-[4-(3,3,5,5-tétraméthylcyclohexyl)phényl]éthanone (*composé V.1*) en solution dans du dichlorométhane. Après 2 heures d'agitation à 0°C on fait barboter dans la réaction 17,1 ml de chlore gazeux (d = 1,565, mesuré à l'état liquide à -78°C). Après retour à température ambiante, on additionne au mélange réactionnel un mélange eau / glace. On extrait au dichlorométhane, décante, sèche la phase organique sur sulfate de magnésium et concentre sous pression réduite. On purifie sur une colonne de gel de silice en éluant avec un mélange cyclohexane / dichlorométhane, 7/3 (v/v) ; rdt = 74% ; F = 64°C.

On isole également les composés dichlorés :

### 1-[3,5-Dichloro-4-(3,3,5,5-tétraméthylcyclohexyl)phényl]éthanone, composé V.5

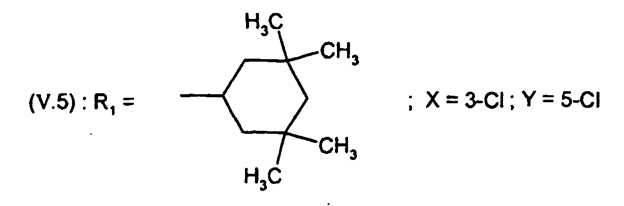

¹H RMN : 7,9 (s,1H) ; 7,8 (s,1H) ; 3,9 (m,1H) ; 2,5 (s,3H) ; 2,1 (m,2H) ; 1,2 (m,4H) ; 1,0 (s,6H) ; 0,9 (s,6H).

### 1-[3,6-Dichloro-4-(3,3,5,5-tétraméthylcyclohexyl)phényl]éthanone, composé V.6

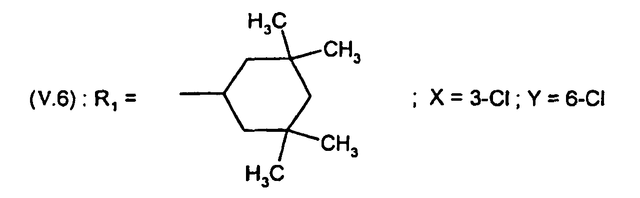

¹H RMN : 7,6 (s, 1H) ; 7,2 (s, 1H) ; 3,3 (m, 1H) ; 2,6 (s,3H) ; 1,5 (m,2H) ; 1,2 (m,4H) ; 1,1 (s,6H) ; 0,9 (s,6H).

Selon le mode opératoire décrit pour le *composé V.4*., on isole les composés suivants

### 1-[3-Chloro-4-(3,3-diméthylcyclohexyl)phényl]éthanone, composé V.7

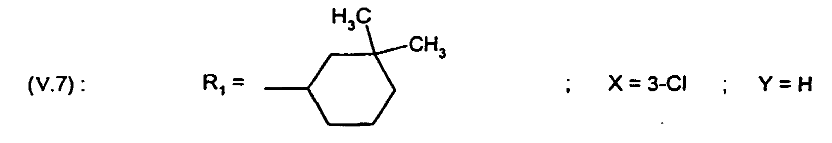

¹H RMN : 7,9 (s,1H) ; 7,8 (d,1H) ; 7,4 (d,1H) ; 3,1 (m,1H) ; 2,5 (s,3H) ; 1,8-1,1 (m,8H) ; 0,9 (s,3H) ; 0,8 (s,3H).

### 1-(3-Chloro-4-tert-butylphényl)éthanone, composé V.8

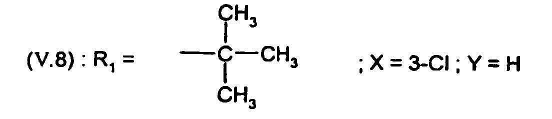

¹H RMN : 7,8 (s,1H) ; 7,7 (d,1H) ; 7,5 (d,1H) ; 2,5 (s,3H) ; 1,4 (s,9H).

### 1-(3,5-Chloro-4-cyclohexylphényl)éthanone, composé V.9

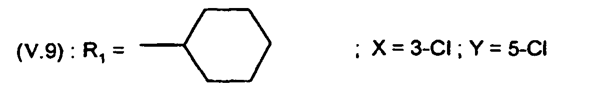

### PREPARATION 5

### 1-[(3-Chloro-4-hydroxy)phényl]éthanone, composé VII.1

(VII.1) : X = 3-Cl ; Y = H

Sous atmosphère inerte, à 63,5 ml de 2-chloro-1-méthoxybenzène dans 500 ml de 1,2-dichloroéthane, on additionne 167 g de trichlorure d'aluminium puis on ajoute goutte à goutte 167 g de chlorure d'acétyle en solution dans 200 ml de 1,2-dichloroéthane. On chauffe le mélange réactionnel à 45°C pendant 48 heures. On verse le mélange réactionnel sur un mélange eau/glace, extrait au dichlorométhane, évapore les solvants sous pression réduite et purifie le résidu obtenu par chromatographie sur une colonne de gel de silice en éluant avec un mélange cyclohexane/acétate d'éthyle, 90/10 (v/v). On recristallise le *composé VII. 1* dans le cyclohexane ; F = 107°C.

### PREPARATION 6

### 1-Prop-2-ynylazépane, composé 4.1

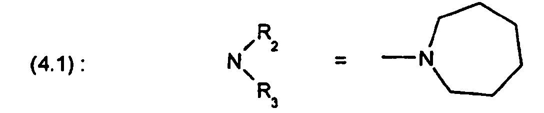

A 20,8 ml d'hexaméthylèneamine et 27,9 g de carbonate de potassium dans 300 ml d'acétonitrile, on additionne goutte à goutte 18,8 ml d'une solution de 3-bromopropyne à 80% dans le toluène. On chauffe le mélange réactionnel à 50°C pendant 12 heures et 6 heures à 80°C. On filtre, évapore les solvants sous pression réduite. Le *composé 4.1* est purifié par distillation ; T_{eb} = 61 °C sous une pression de 26,7 Pa.
¹ H RMN : 3,3 (s,2H) ; 3,0 (s,1H) ; 2,5 (m,4H) ; 1,5 (m,8H).

De la même manière on prépare:

### 1-Prop-2-ynylazocane, composé 4.2

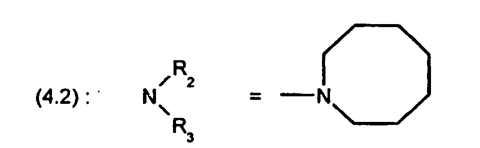

¹H RMN : 3,3(s,2H) ; 3,0(s,1H) ; 2,5(m,4H) ; 1,5(m,10H).

### 1-Prop-2-ynylpipéridine, composé 4.3

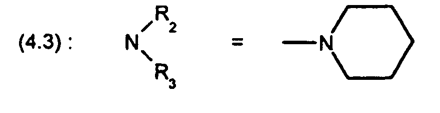

¹H RMN : 3,2(s,2H) ; 3,1(s,1H) ; 2,3(m,4H) ; 1,5(m,2H) ; 1,3(m,4H).

### PREPARATION 7

### 4-Acétyl-2-chlorophényltrifluorométhanesulfonate, composé Va.1

(Va.1): X = 3-Cl ; Y = H ; Z = OTf

A 0°C, à 26,7 g de 1-[(3-chloro-4-hydroxy)phényl]éthanone (composé *VII.1*) dans 700 ml de pyridine, on additionne goutte à goutte 26,2 ml d'anhydride triflique. On agite le mélange réactionnel à 0°C pendant 36 heures, évapore les solvants sous pression réduite et reprend le résidu avec une solution 0,1 N d'acide chlorhydrique dans le dichlorométhane. On décante, sèche les phases organiques sur sulfate de magnésium et évapore les solvants sous pression réduite. Le résidu obtenu est purifié par chromatographie sur une colonne de gel de silice en éluant avec un mélange cyclohexane/acétate d'éthyle , 95/5 (v/v).
¹H RMN : 8,2 (s,1H) ; 8,0 (d,1H) ; 7,8 (d,1H).

De la même manière on prépare les composés suivants :

### 4-Acétyl-2,6-dichlorophényltrifluorométhanesulfonate, composé va.2

(Va.2) : X = 3-Cl ; Y = 6-Cl ; Z = OTf

¹H RMN : 8,2 (s,2H) ; 2,6 (s,3H).

### 4-Bromo-2-chlorophényltrifluorométhanesulfonate , composé IIIa.1 à partir du 4-bromo-2-chlorophénol.

(IIIa. 1) : X = 3-Cl ; Y = H

¹H RMN : 8,1 (s,1H) ; 7,7 (d,1H) ; 7,6 (d,1H).

### 4-Bromo-3-chlorophényltrifluorométhanesulfonate , composé IIIa.2

(IIIa.2) : X = 2-Cl ; Y =H

¹H RMN : 8,0(m,2H) ; 7,5(d,1H)

### 4-Bromo-2-méthylphényltrifluorométhanesulfonate , composé IIIa.3

(IIIa.3) : X = 2-CH₃ ; Y = H

¹H RMN : 7,7(s,1H) ; 7,6(d,1H) ; 7,3(d,1H) ; 2,3(s,3H).

### 4-Bromophényltrifluorométhanesulfonate, composé IIIa.4

(*IIIa*.4) : X = Y = H

¹H RMN : 7,8(d,2H) ; 7,4(d,2H).

### PREPARATION 8

### 4-[3-(1-Azépanyl)prop-1-ynyl]-2-chlorophényltrifluorométhanesulfonate, composé Ia.1

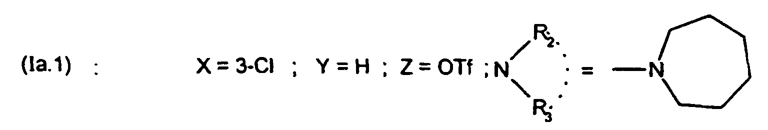

Sous atmosphère inerte, à 4 g de 4-bromo-2-chlorophényltrifluorométhanesulfonate *(composé IIIa.1),* 0,062 g d'iodure de cuivre, 10 ml de pyridine et 20 ml de triéthylamine, on additionne 1,96 g de 1-prop-2-ynylazépane (*composé 4.1*) puis 0,457 g du catalyseur dichlorobis(triphénylphosphine)palladium. On chauffe le mélange réationnel à reflux pendant 2 heures, évapore les solvants sous pression réduite, reprend le résidu obtenu au dichlorométhane, lave à l'eau et sèche sur sulfate de magnésium. Après évaporation des solvants sous pression réduite, le résidu obtenu est purifié par chromatographie sur une colonne de gel de silice en éluant avec un mélange cyclohexane/acétate d'éthyle 80/20 ; F =192°C.
¹H RMN : 7,8 (s,1H) ; 7,5 (m,2H) ; 3,6 (s,2H) ; 2,6 (m,4H) ; 1,5 (m,8H).

De la même manière, on prépare les composés suivants :

### 4-[3-(1-Azépanyl)prop-1-ynyl]-3-chlorophényltrifluorométhanesulfonate, composé Ia.2

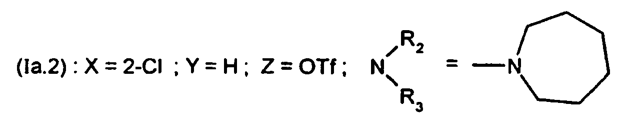

¹H RMN : 7,7(d,1H) ; 7,6(s,1H) ; 7,3(d,1H) ; 3,5(s,2H) ; 2,6(m,4H) ; 1,5(m,8H).

### 4-[3-(1-Azépanyl)prop-1-ynyl]-2-méthylphényltrifluorométhanesulfonate, composé Ia.3

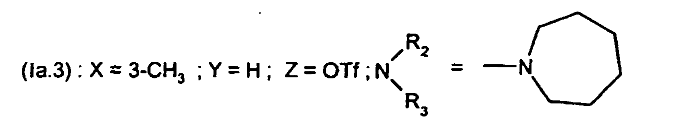

¹H RMN : 7,5(s,1H) ; 7,4(m,2H) ; 3,5(s,2H) ; 2,6(m,4H) ; 2,3(s,3H) ; 1,5(m,8H).

### 4-[3-(1-Azocanyl)prop-1-ynyl]-2-chlorophényltrifluorométhanesulfonate, composé Ia.4

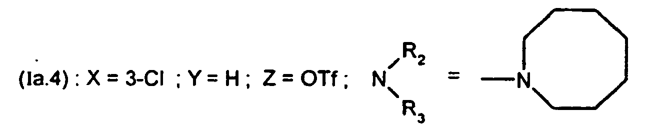

¹H RMN : 7,8(s,1H) ; 7,5(m,2H) ; 3,6(s,2H) ; 2,6(m,4H) ; 1,5(m,10H).

### 4-[3-(1-Pipéridinyl)prop-1-ynyl]-2-chlorophényltrifluorométhanesulfonate, composé Ia.5

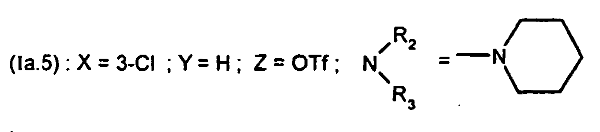

¹H RMN : 7,8(s,1H) ; 7,6(m,2H) ; 3,5(s,2H) ; 2,4(m,4H) ; 1,8-1,5(m,6H).

### 4-[3-(1-Azépanyl)prop-1-ynyl]phényltrifluorométanesulfonate, composé Ia.6

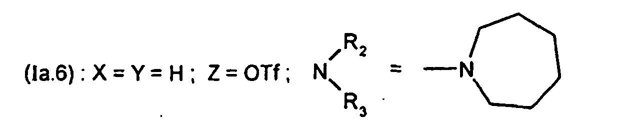

### PREPARATION 9

### 1-[3-Chloro-4-(4-fluorophényl)phényl]éthanone, composé V.10

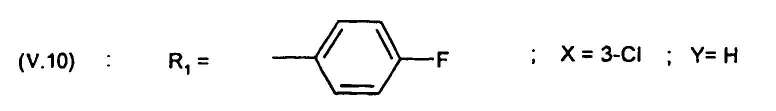

Sous atmosphère inerte, on agite à 60°C pendant 8 heures 19,7 g de 4-acétyl-2-chlorophényltrifluorométhanesulfonate (*composé Va.1*), 10 g d'acide 4-fluorobenzèneboronique, 2 g de tétrakis(triphénylphosphine)palladium, 17,9 g de carbonate de sodium dans 84,5 ml d'eau, 591 ml de toluène, 200 ml d'éthanol et 5,51 g de chlorure de lithium. On agite ensuite le mélange réactionnel pendant 12 heures à température ambiante. On filtre, évapore lés solvants du filtrat sous pression réduite. Le résidu obtenu est purifié par chromatographie sur une colonne de gel de silice en éluant avec un mélange cyclohexane/acétate d'éthyle, 97/3 (v/v) ; rdt = 94%.
¹H RMN : 8,0 (1H,s) ; 7,9 (1H,d) ; 7,5 (3H,m) ; 7,3 (2H,m) ; 2,6 (3H,s).

De la même manière, on prépare les *composés V.11* à *V.15* présentés dans le TABLEAU 1 suivant :

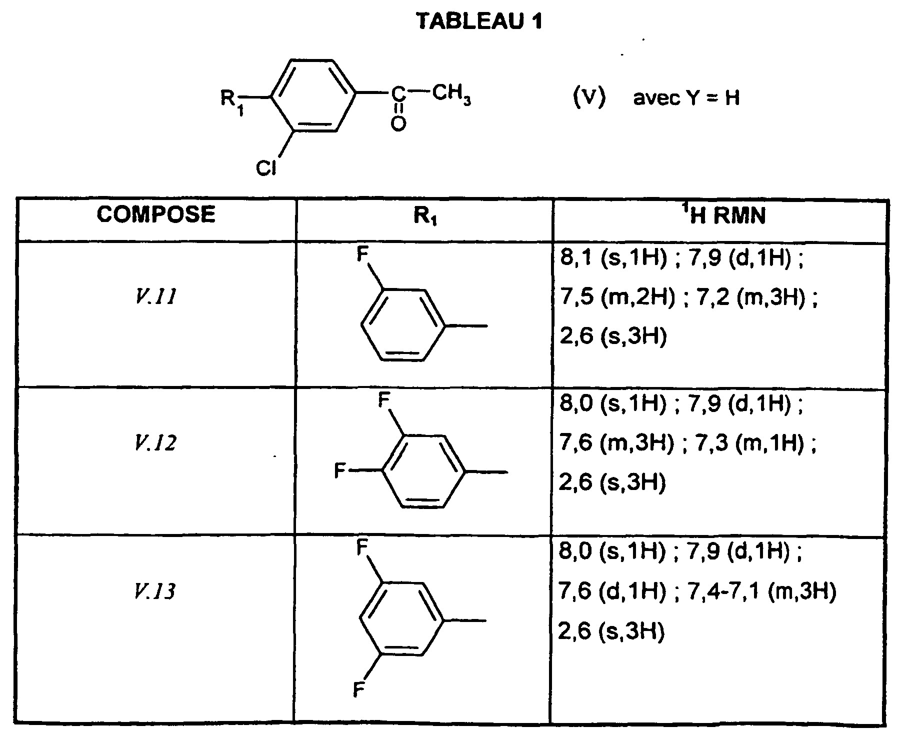

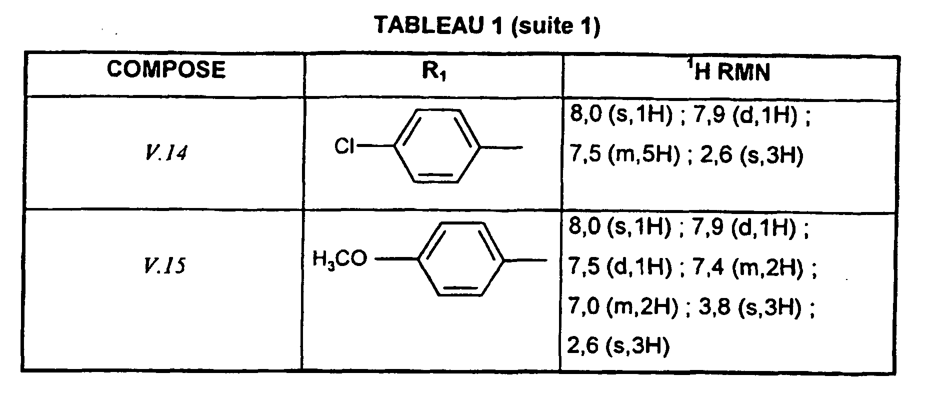

### 1-(2,6-Dichlorobiphényl-4-yl)éthanone, composé V.16

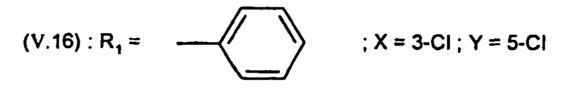

¹H RMN : 8,0 (s,2H) ; 7,4 (m,3H) ; 7,2 (m,2H) ; 2,6 (s,3H).

### 1-(2,6-Dichloro-4'-fluorobiphényl-4-yl)éthanone, composé V.17

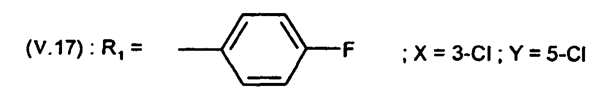

¹H RMN : 8,0 (s,2H) ; 7,3 (m,4H) ; 2,6 (s,3H).

### PREPARATION 10

### 3-Chloro-3-[3-chloro-4-(3,3,5,5-tétraméthylcyclohexyl)phényl]propénal, composé IV.1

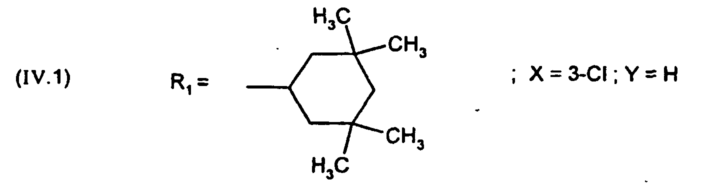

A une température comprise entre -5 et 2°C, à une solution de 3,72 ml de diméthylformamide et de 20 ml de dichlorométhane anhydre, on ajoute goutte à goutte 3,51 ml de chlorure d'oxalyle, puis agite le mélange réactionnel pendant 30 minutes à température ambiante. On additionne alors rapidement 3,92 g de 1-[3-chloro-4-(3,3,5,5-tétraméthylcyclohexyl)phényl]éthanone (*composé V.6*) en solution dans 10 ml de dichlorométhane, puis on agite le mélange réactionnel à température ambiante pendant 12 heures. On verse le mélange réactionnel sur un mélange eau/glace, puis on ajoute 20 ml d'une solution aqueuse d'éthanoate de sodium à 2,84 M. On lave avec 50 ml d'une solution d'hydrogénocarbonate de sodium et 50 ml d'eau, décante, sèche la phase organique sur sulfate de magnésium et évapore les solvants sous pression réduite. On purifie l'huile obtenue par chromatographie sur une colonne de gel de silice en éluant avec un mélange cyclohexane/acétate d'éthyle, 97/3 (v/v).
¹H RMN : 10,2 (d,1H) ; 7,7 (s,1H) ; 7,5 (d,1H) ; 7,3 (d,1H) ; 6,6 (d,1H) ; 3,4 (m,1H) ; 1,5 (m,2H) ; 1,3 (m,4H) ; 1,1 (s,6H) ; 0,9 (s,6H).

De la même manière, on prépare les composés *IV.2* à *IV.18* présentés dans les TABLEAUX 2 et 3 ci-après :

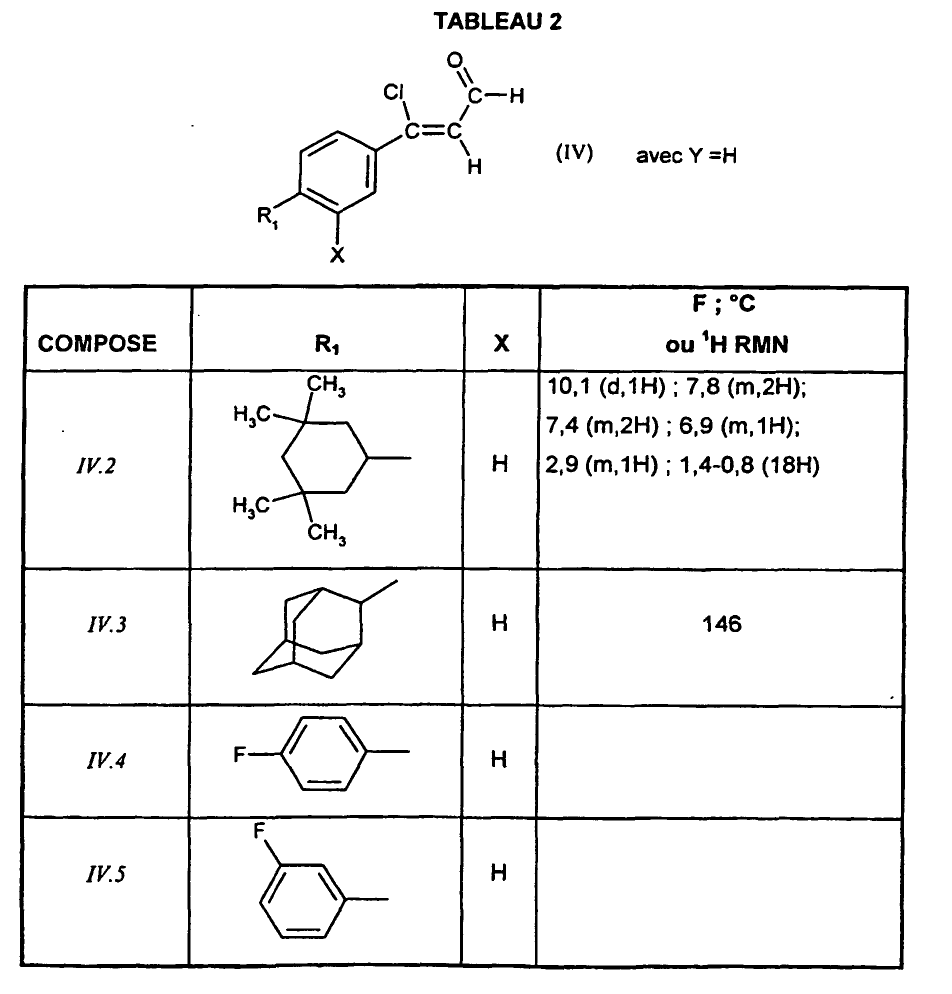

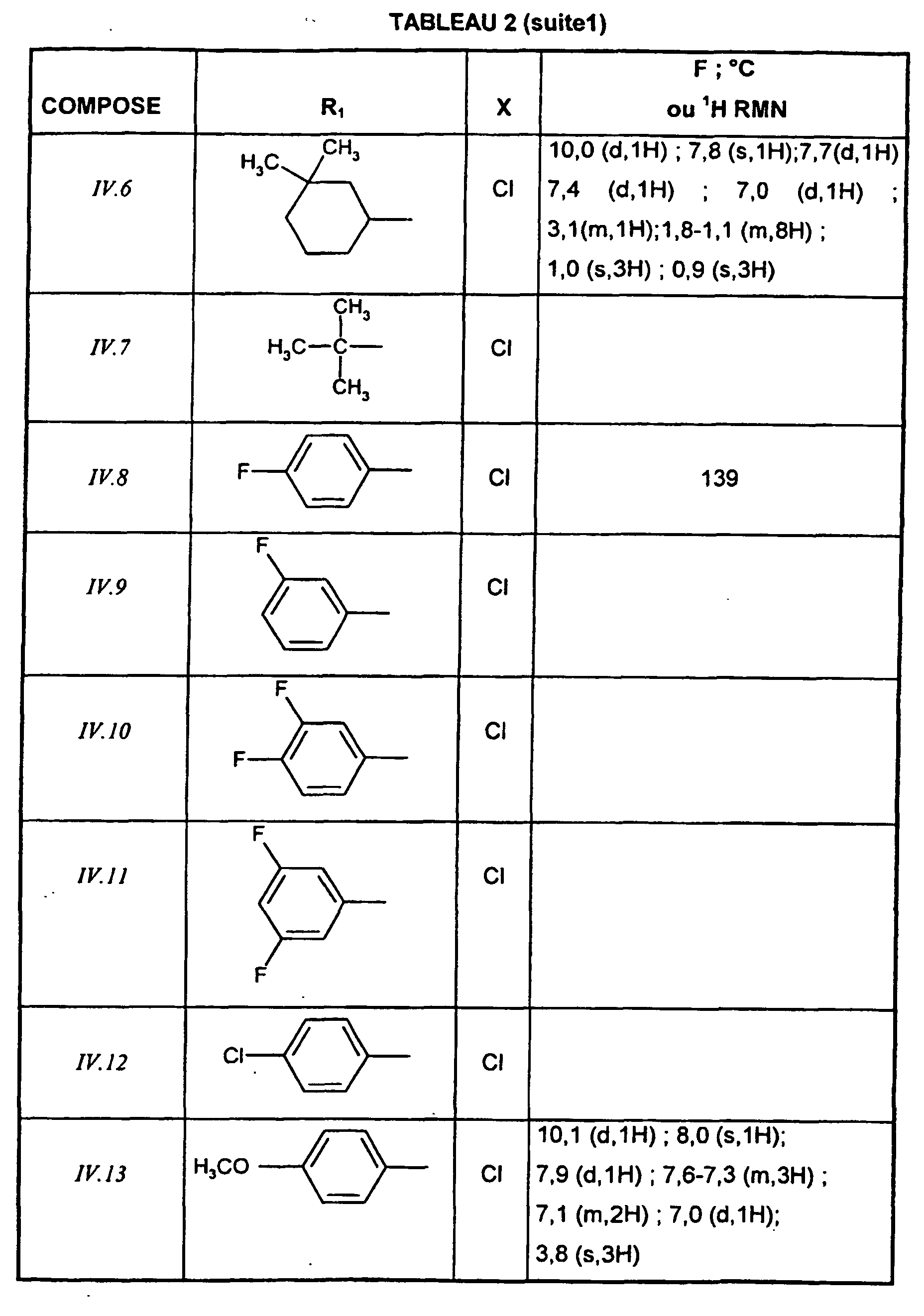

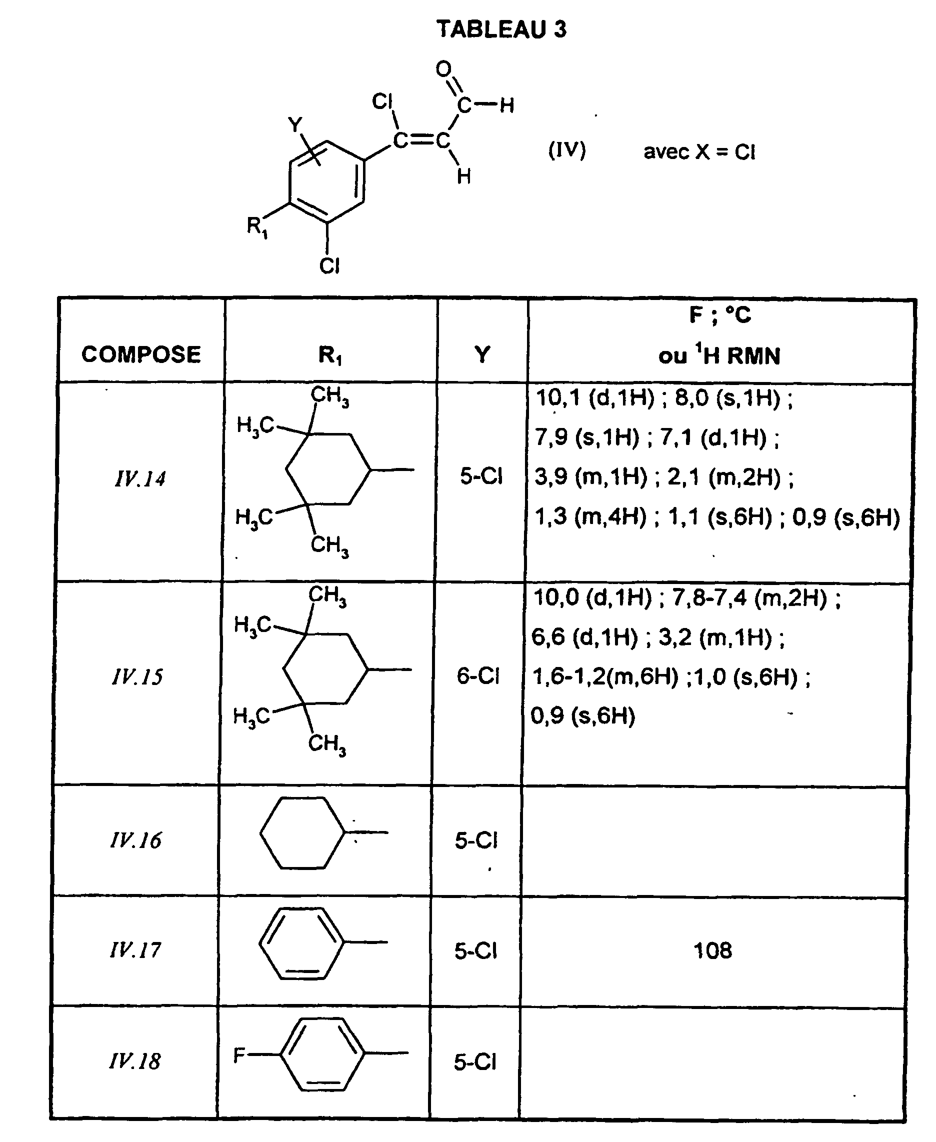

### PREPARATION 11

### 3-Chloro-4-(3,3,5,5-tétraméthylcyclohexyl)phényléthyne, composé II.1.

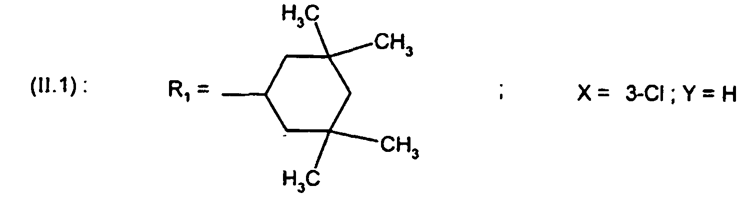

Sous atmosphère inerte et sous forte agitation on dissout 5,3 g d'hydroxyde de sodium dans 150 ml d'eau. On ajoute 80 ml de 1,4-dioxane et chauffe à reflux. On additionne rapidement 15 g de 3-chloro-3-[3-chloro-4-(3,3,5,5-tétraméthylcyclohexyl)phényl] propénal (*composé IV.1*) en solution dans 130 ml de 1,4-dioxane et maintient le mélange réactionnel à reflux pendant 1 heure. Après retour à température ambiante on verse le mélange réactionnel sur un grand volume de dichlorométhane. On décante, sèche la phase organique sur sulfate de magnésium et évapore les solvants sous pression réduite. On purifie par chromatographie sur une colonne de gel de silice en éluant avec du cyclohexane ; rdt = 80%.
¹H RMN : 7,5 (1H,s) ; 7,3 (2H,m) ; 4,2 (1H,s) ; 3,2 (1H,m) ; 1,4 (2H,m) ; 1,2 (4H,m) ; 1,0 (6H,s) ; 0,9 (6H,s).

De la même manière, on prépare les composés *II.2* à *II.16* présentés dans les TABLEAUX 4 et 5 ci-après :

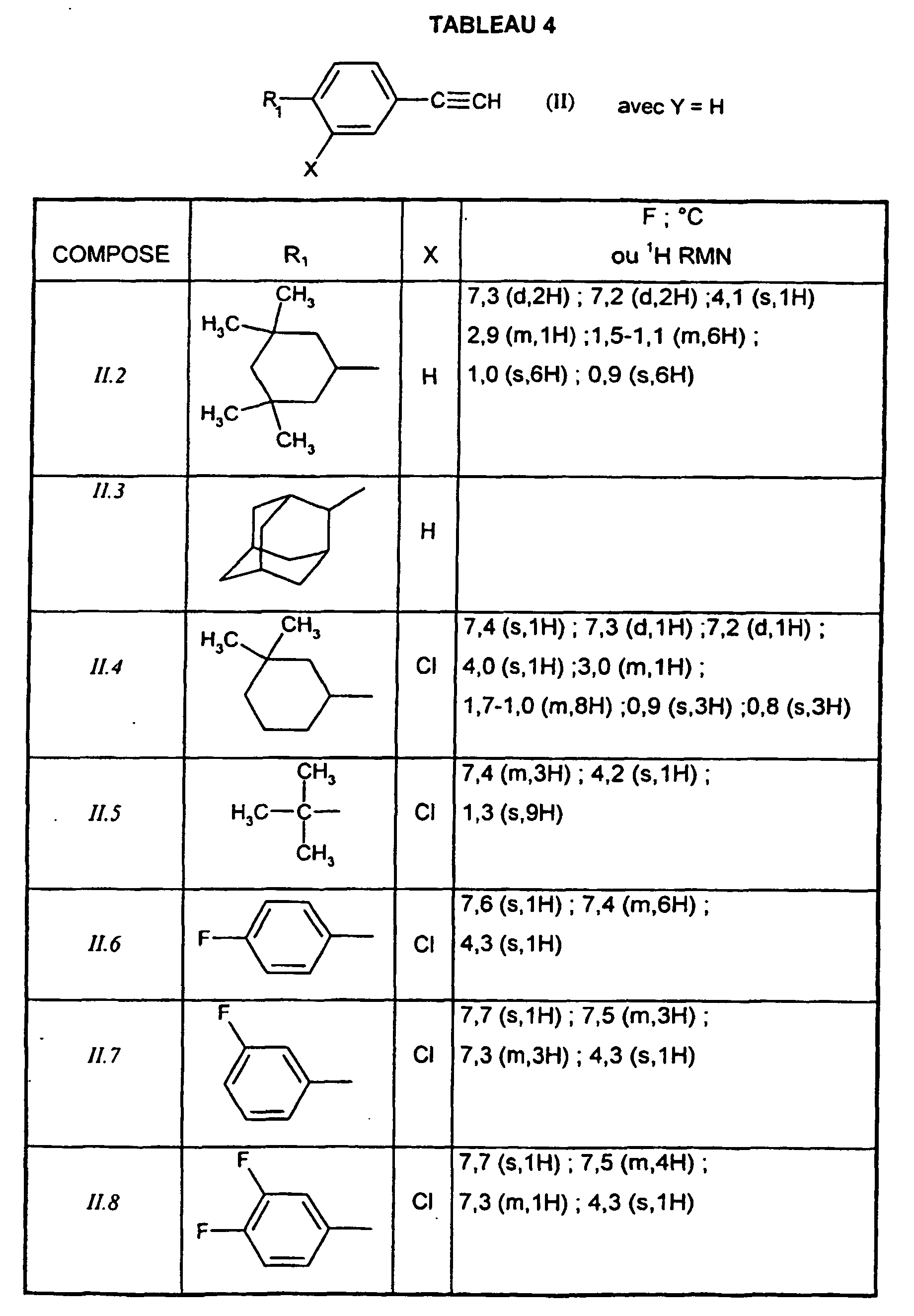

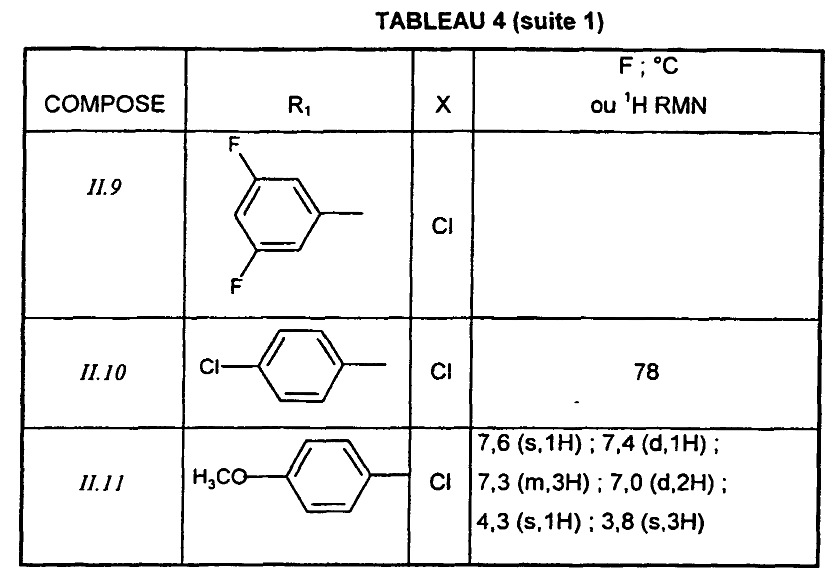

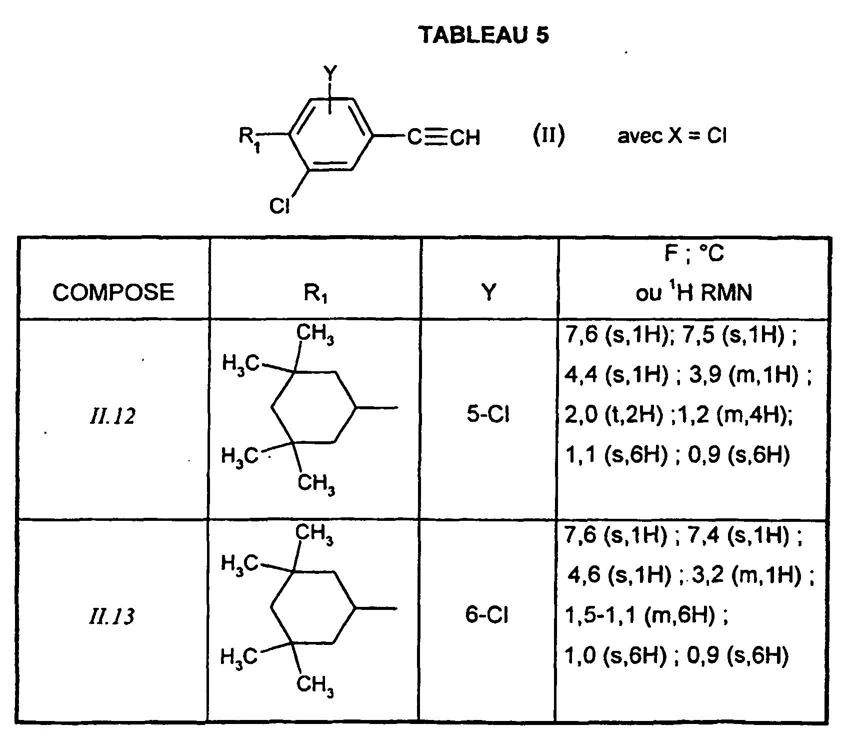

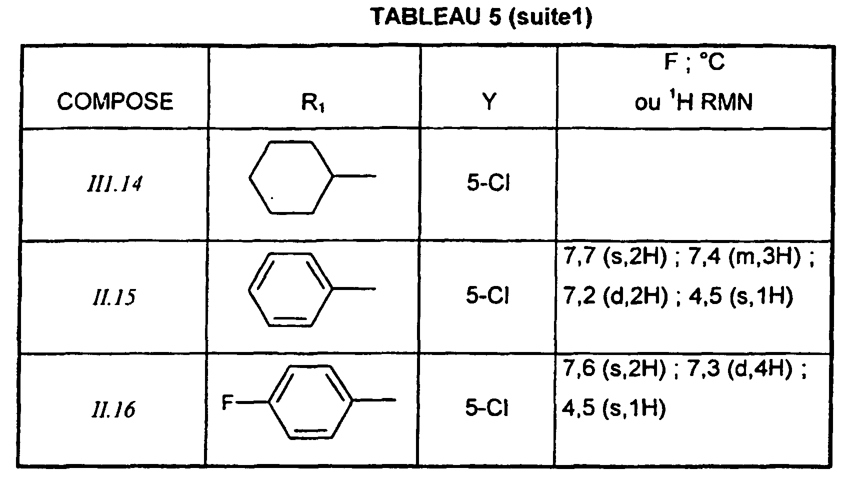

### PREPARATION 12

### Acide 3,5-difluorobenzèneboronique, composé 2.1

A -78°C, à 20 g de 1-bromo-3,5-fluorobenzène dans 300 ml de éther diéthylique, on additionne 91,5 ml de *tert*-butyllithium. On agite le mélange réactionnel pendant 1 heure à -78°C puis additionne 14,2 ml de triméthylborate. On agite le mélange réactionnel pendant 1 heure à -78°C puis pendant 12 heures à température ambiante. On additionne 200 ml d'une solution aqueuse 1 N d'acide chlorhydrique. On extrait au éther diéthylique, lave la phase organique avec une solution aqueuse saturée en hydrogènocarbonate de sodium, la sèche sur sulfate de magnésium et évapore les solvants sous pression réduite. On reprend le résidu au cyclohexane et isole le précipité obtenu par filtration.
¹H RMN :7,4 (3H,m) ; 7,2 (2H,m).

### PREPARATION 13

### 4-Bromo-3-chloroacétophénone, composé Va.3

(Va.3) ; X = 3-Cl ; Y = H ; Z = Br

A 0°C, à 133,34 g de chlorure d'aluminium dans 600 ml de dichlorométhane, on additionne goutte à goutte une solution de 100 g de 4-bromoacétophénone dans 250 ml de dichlorométhane. Après 2 heures d'agitation à 0°C. on fait barbotter dans le milieu à 0°C 28,3 ml de chlore préalablement congelé (-75°C). On agite à température ambiante pendant 12 heures puis hydrolyse le mélange réactionnel. On décante, extrait au dichlorométhane, sèche les phases organiques sur sulfate de magnésium et évapore les solvants sous pression réduite. On recristallise le résidu obtenu dans l'hexane ; rdt = 57% ; F = 80°C.

### PREPARATION 14

### 3-Chloro-3-(4-bromo-3-chlorophényl)propénal, composé IVa.1

(IVa.1) : X = 3-Cl ; Y = H ; Z = Br

A une température comprise entre 3 et 6°C, on additionne sous forte agitation 15,08 ml de chlorure d'oxalyle à 16 ml de diméthylformamide dans 200 ml de dichlorométhane. Après retour à température ambiante, on agite pendant 30 minutes puis ajoute une solution de 13,4 g de 4-bromo-3-chloroacétophénone (*composé Va.3*) dans 40 ml de dichlorométhane. On agite le mélange réactionnel pendant 12 heures à température ambiante, hydrolyse le mélange réactionnel par ajout d'une solution de 18,9 g d'acétate de sodium dans 50 ml d'eau. Après 30 minutes d'agitation à température ambiante, on décante, extrait au dichlorométhane, sèche les phases organiques sur sulfate de magnésium et évapore les solvants sous pression réduite. Le résidu obtenu est recristallisé dans le cyclohexane ; rdt = 87% ; F = 134°C.

### PREPARATION 15

### 1-[3-(4-Bromo-3-chlorophényl)prop-2-ynyl]azépane, composé Ia.7

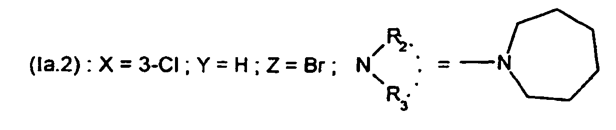

a) 1-Bromo-2-chloro-4-éthynylbenzène, *composé IIa.1*
   Sous atmosphère inerte, on dissout 6,9 g d'hydroxyde de sodium dans 220 ml d'eau, on additionne 100 ml de 1,4-dioxane et chauffe le mélange réactionnel à 75°C. On additionne 16 g de 3-chloro-3-(4-bromo-3-chlorophényl)propénal (*composé IVa.1)* en solution dans 400 ml de 1,4-dioxane et agite le mélange réactionnel pendant 30 minutes à 85°C. On laisse revenir le mélange réactionnel à température ambiante puis additionne 1300 ml de dichlorométhane. On décante, lave la phase organique à l'eau et la sèche sur sulfate de magnésium et évapore les solvants. Le composé obtenu est directement engagé dans l'étape suivante.
b)1-[3-(4-Bromo-3-chlorophényl)prop-2-ynyl]azépane, *composé Ia.7*
   On ajoute 2,53 ml d'une solution aqueuse à 36% de formaldéhyde sur 2,46 ml d'hexaméthylèneimine dans 40 ml de 1,2-diméthoxyéthane On additionne cette solution à 4,28 g du composé obtenu précédemment en présence de 0,17 g de chlorure de cuivre II, dihydraté dans 120 ml de 1,2-diméthoxyéthane. On agite le mélange réactionnel pendant 1 heure à reflux, évapore les solvants sous pression réduite puis purifie le résidu obtenu par chromatographie sur une colonne de gel de silice en éluant avec un mélange cyclohexane/acétate d'éthyle variant de 90/10 à 80/20 (v/v) ; rdt = 82%.
   ¹H RMN : 7,7 (d,1H) ; 7,6 (s,1H) ; 7,2 (d,1H) ; 3,5 (s,2H) ; 2,6 (m,4H) ; 1,5 (m,8H).

### PREPARATION 16

### 2-{2-Chloro-4-[3-(1-azépanyl)prop-1-ynyl]phényl}adamantan-2-ol, composé I'.1

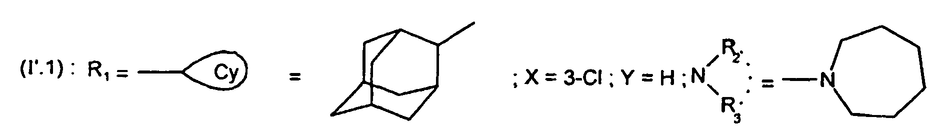

A -78°C, à 3,1 g de 1-[3-(4-bromo-3-chlorophényl)prop-2-ynyl]azépane (*composé Ia.7*) dans 50 ml de éther diéthylique, on additionne 5,6 ml d'une solution de *n*-butyllithium à 15% dans l'hexane et maintient l'agitation à -75°C pendant 1 heure. Toujours à -78°C, on additionne 1,38 g d'amandatan-2-one dans 25 ml de éther diéthylique puis agite le mélange réactionnel pendant 1 heure à -78°C.

On laisse le mélange réactionnel revenir à température ambiante puis on additionne un mélange eau/glace. On décante, extrait au éther diéthylique, sèche les phases organiques sur sulfate de sodium et évapore les solvants sous pression réduite. Le résidu obtenu est purifié par chromatographie sur une colonne de gel de silice en éluant avec un mélange cyclohexane/acétate d'éthyle 85/15 (v/v) ; rdt = 73% ; F = 95°C.

### PREPARATION 17

### 4,4-Diméthylcyclohexanone, composé 3.1

a) 4,4-Diméthylcyclohex-2-ènone
   A 81 ml de but-3-èn-2-one et 88 ml de 2-méthylpropionaldéhyde dans 450 ml de benzène, on additionne à température ambiante 1 ml d'acide sulfurique concentré, puis chauffe le mélange réactionnel à reflux pendant 13 heures pour éliminer l'eau par entraînement azéotropique. Après retour à température ambiante, le mélange réactionnel est lavé avec une solution aqueuse saturée en bicarbonate de sodium, puis à l'eau. La phase organique est séchée sur sulfate de magnésium et les solvants évaporés sous pression réduite. Après distillation, on isole 31,1 g du composé attendu ; T_{Eb} = 78 °C (sous une pression de 22 mbar).
b) 31,1 g de 4,4-diméthylcyclohex-2-ènone dans 100 ml de pentane, sont hydrogénés en autoclave sous une pression de 5 bar en présence de 1,6 g de palladium sur charbon à 5%. Le mélange réactionnel est filtré et les solvants évaporés sous pression réduite.

### PREPARATION18

### 2-Chloro-4-(4,4-diméthylcyclohexyl)phénol, composé IX.1

a) 2-Chloro-4-(1-hydroxy-4,4-diméthylcyclohexyl)phénol
   A 15,1 g de 4-bromo-2-chlorophénol dans 150 ml de tétrahydrofurane, on additionne à -78°C 100 ml d'une solution 1,6M de butyllithium dans l'hexane, agite le mélange réactionnel pendant 1 heure à -78°C. On additionne 10,1 g du 4,4-diméthylcyclohexanone (*composé 3.1*) et agite à nouveau le mélange réactionnel pendant 30 minutes à -78°C puis pendant 12 heures à température ambiante. Le mélange réactionnel est hydrolysé par une solution d'acide chlorhydrique 1N et extrait avec de l'acétate d'éthyle. La phase organique est séchée sur sulfate de magnésium et les solvants évaporés sous pression réduite. Le solide obtenu est purifié par chromatographie sur une colonne de gel de silice en éluant avec un mélange cyclohexane/acétate d'éthyle variant de 98/2 à 90/10 (v/v). On obtient 11,8 g de solide. ¹H RMN : 7,4(s,1H) ; 7,2(d,2H) ; 6,9(d,2H) ; 4,5(s,1H) ; 1,9-1,1(m,8H) ; 0,9(s,6H)
b) A 11,8 g du 2-chloro-4-(1-hydroxy-4,4-diméthylcyclohexyl)phénol dans 200 ml d'acide acétique, on additionne 50 ml d'une solution aqueuse d'acide iodhydrique à 57%. Le mélange réactionnel est chauffé à reflux pendant 3 heures, les solvants évaporés sous pression réduite. On ajoute une solution aqueuse à 40% d'hydroxyde de sodium, une solution aqueuse de carbonate de sodium, puis une solution aqueuse d'hydrogénosulfate de sodium et extrait avec de l'éther diéthylique. La phase organique est séchée sur sulfate de magnésium et les solvants évaporés sous pression réduite. On purifie le composé obtenu par chromatographie sur colonne de gel de silice en éluant avec un mélange cyclohexane/acétate d'éthyle 95/5 (v/v).
   ¹H RMN : 9,8(s,1H) ; 7,1(s,1H) ; 7(d,1H) ; 6,9(d,1H) ; 1,9(m,1H) ; 1,6-1,2(m,8H) ; 0,9(s,6H)

### PREPARATION 19

### 2-Chloro-4-(4,4-diméthylcyclohexyl)phényltrifluorométhanesulfonate, composé III.1

A 9,7 g du 2-chloro-4-(4,4-diméthylcyclohexyl)phénol (*composé IX.1*) dans 60 ml de pyridine, on additionne à 5°C 8,2 ml d'anhydride triflique et laisse le mélange réactionnel pendant 30 minutes à 0°C, puis on agite le mélange réactionnel à température ambiante pendant 12 heures. Le mélange réactionnel est hydrolysé puis extrait au dichlorométhane. La phase organique est séchée sur sulfate de magnésium et les solvants évaporés sous pression réduite. Le résidu obtenu est repris avec du toluène puis les solvants sont évaporés sous pression réduite. Le résidu obtenu est purifié par chromatographie sur une colonne de gel de silice en éluant avec un mélange cyclohexane /acétate d'éthyle variant de 100/0 à 99/1 (v/v). On obtient 15 g du composé.
¹H RMN : 7,7(s,1H) ; 7,5(d,1H) ; 7,4(d,1H) ; 2,5(m,1H) ; 1,6-1,2(m,8H) ; 0,92(s,3H) ; 0,86(s,3H).

### EXEMPLE 1

### Chlorhydrate de 1-{3-[3-chloro-4-(3,3,5,5-tétraméthylcyclohexyl)phényl]prop-2-ynyl}azépane.

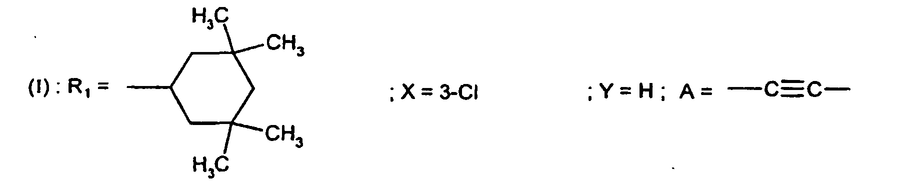

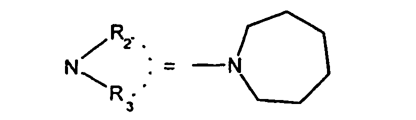

Sous atmosphère inerte, à 2,57 g de 3-chloro-4-(3,3,5,5-tétraméthylcyclohexyl) phényléthyne (*composé II.1*) dans 20 ml de 1,2-diméthoxyéthane (DME), on additionne 0,08 g de chlorure de cuivre II dihydraté. On ajoute ensuite rapidement une solution de 1,19 ml de formaldéhyde et 1,162 ml d'hexaméthylèneimine dans 10 ml de DME. On chauffe le mélange réactionnel à reflux pendant une heure. Après retour à température ambiante, on évapore les solvants sous pression réduite. On reprend le résidu avec du éther diéthylique et fait barboter de l'acide chlorhydrique gazeux sous forte agitation. On isole le précipité obtenu par filtration. Ce précipité est séché sous pression réduite puis recristallisé dans le toluène ; rdt = 75%; F = 187°C (HCl).

De la même manière, on prépare les EXEMPLES 2 à 16 présentés dans les TABLEAUX 6 et 7 ci-après :

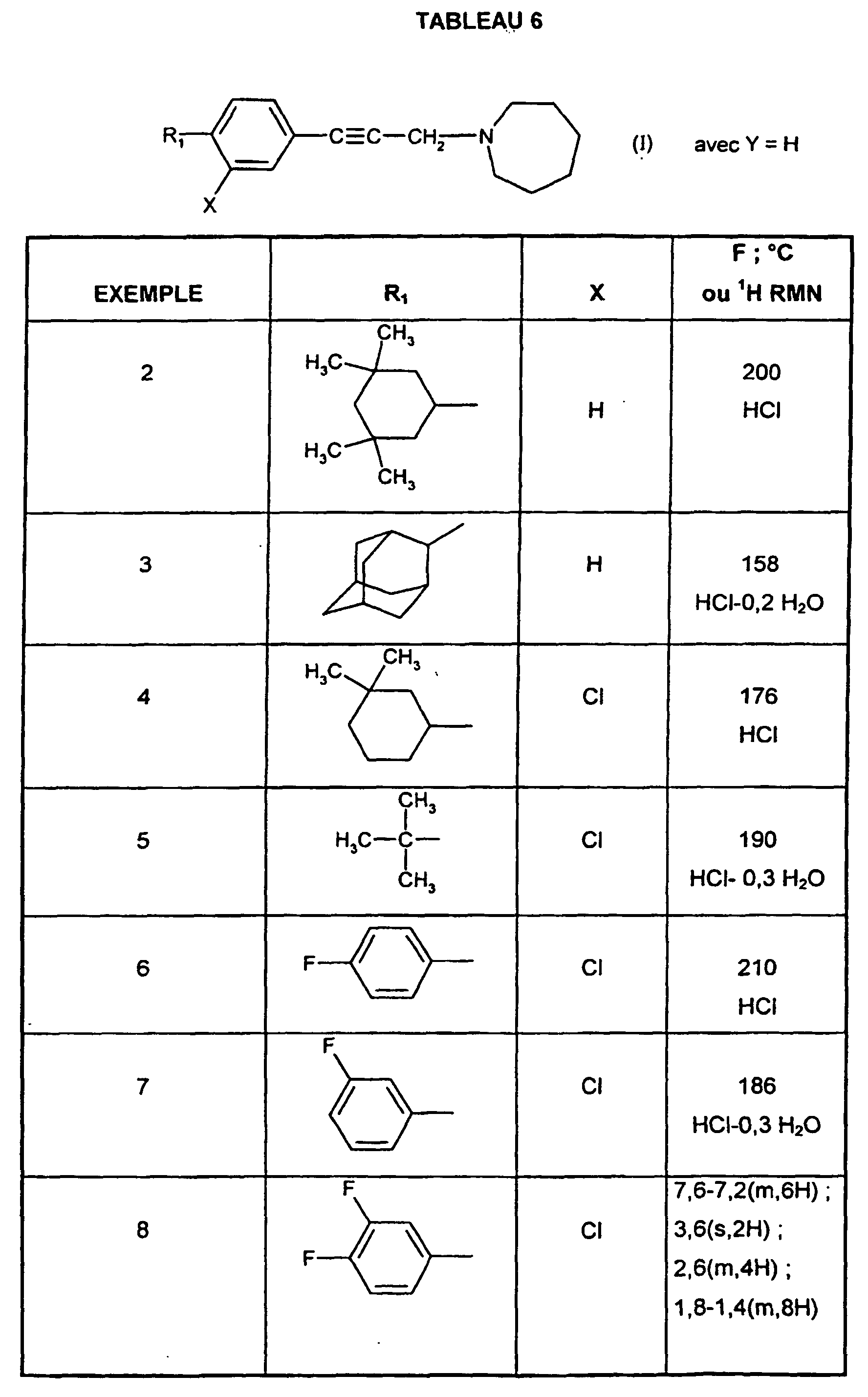

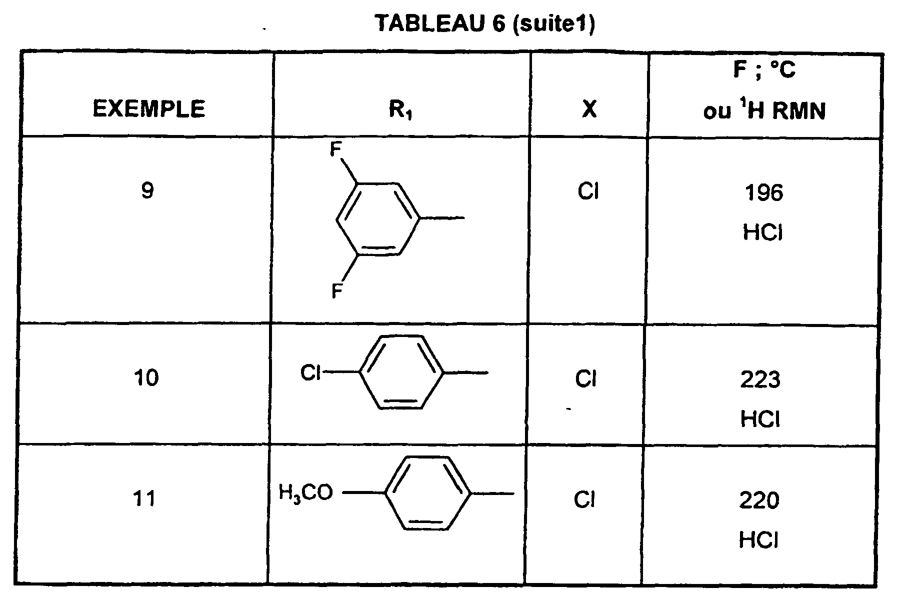

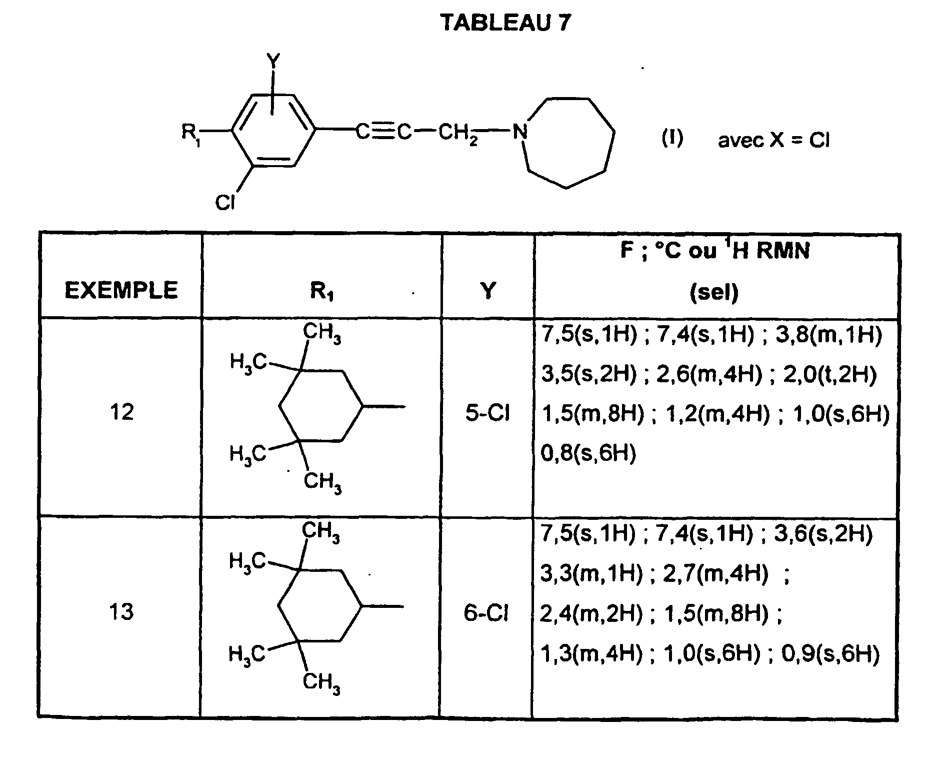

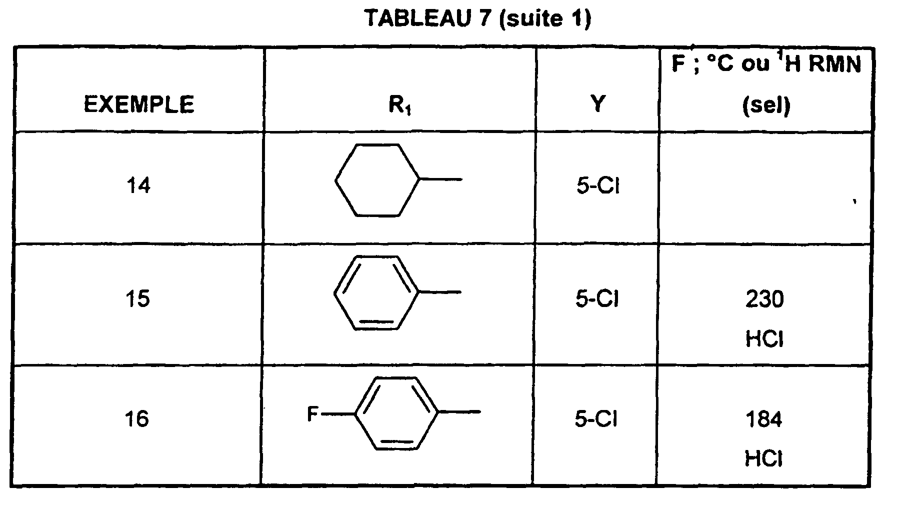

### EXEMPLE 17

### Chlorhydrate de 1-[3-(4-adamantan-2-yl-3-chlorophényl)prop-2-ynyl]azépane.

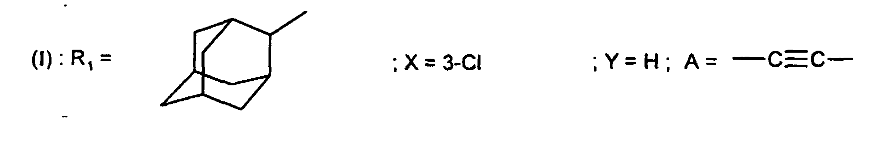

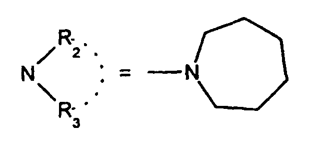

Sous atmosphère inerte, à 3,68 g de 2-{2-chloro-4-[3-(1-azépanyl)prop-1-ynyl]phényl}adamantan-2-ol (*composé I'.1*) dans 20ml d'acétonitrile et 10 ml de dichlorométhane, on additionne 3,46 g d'iodure de sodium puis 2,35 ml de chlorotriméthylsilane. On agite le mélange réactionnel pendant 2 heures à 30°C puis additionne 1,06 ml d'acide acétique, 10 ml d'acétonitrile puis 1,81 g de zinc en poudre. On chauffe le mélange réactionnel à 80°C pendant 3 heures , laisse revenir à température ambiante, filtre et lave au éther diéthylique. On sèche les phases organiques sur sulfate de sodium puis évapore les solvants sous pression réduite. Le résidu obtenu est purifié par chromatographie sur une colonne de gel de silice en éluant avec un mélange cyclohexane/acétate d'éthyle 87,5/12,5 (v/v) ; F = 218°C (HCl).

### EXEMPLE 18

### Chlorhydrate de 1-[3-(2-chloro-3',5'-difluorobiphényl-4-yl)prop-2-ynyl]azépane.

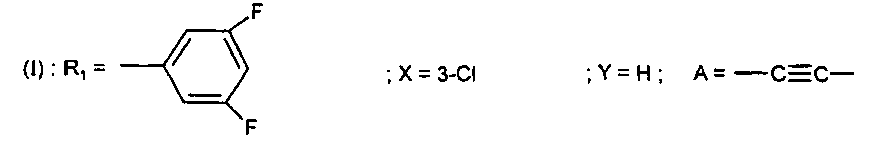

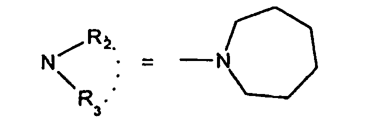

Sous atmosphère inerte, on agite pendant 12 heures à 80°C, 9,5 g de 4-[3-(1-azépanyl)prop-1-ynyl]-2-chlorophényltrifluorométhanesulfonate (*composés Ia.1*), 5 g d'acide 3,5-difluorobenzèneboronique (*composé 2.1*), 31 ml d'une solution aqueuse 2 M de carbonate de sodium, 2,1 g de chlorure de lithium, 300 ml de toluène, 100 ml d'éthanol et 0,7 g de tétrakis(triphénylphosphine)palladium. On évapore les solvants sous pression réduite et purifie le résidu obtenu par chromatographie sur une colonne de gel de silice en éluant avec un mélange toluène/éthanol, 99/1 (v/v). On reprend le composé obtenu avec du éther diéthylique et fait barboter de l'acide chlorhydrique. On filtre et recristallise le précipité obtenu dans le toluène ; F = 196°C (HCl).

De la même manière, on prépare les composés des EXEMPLES 19 à 32 présentés ci-après :

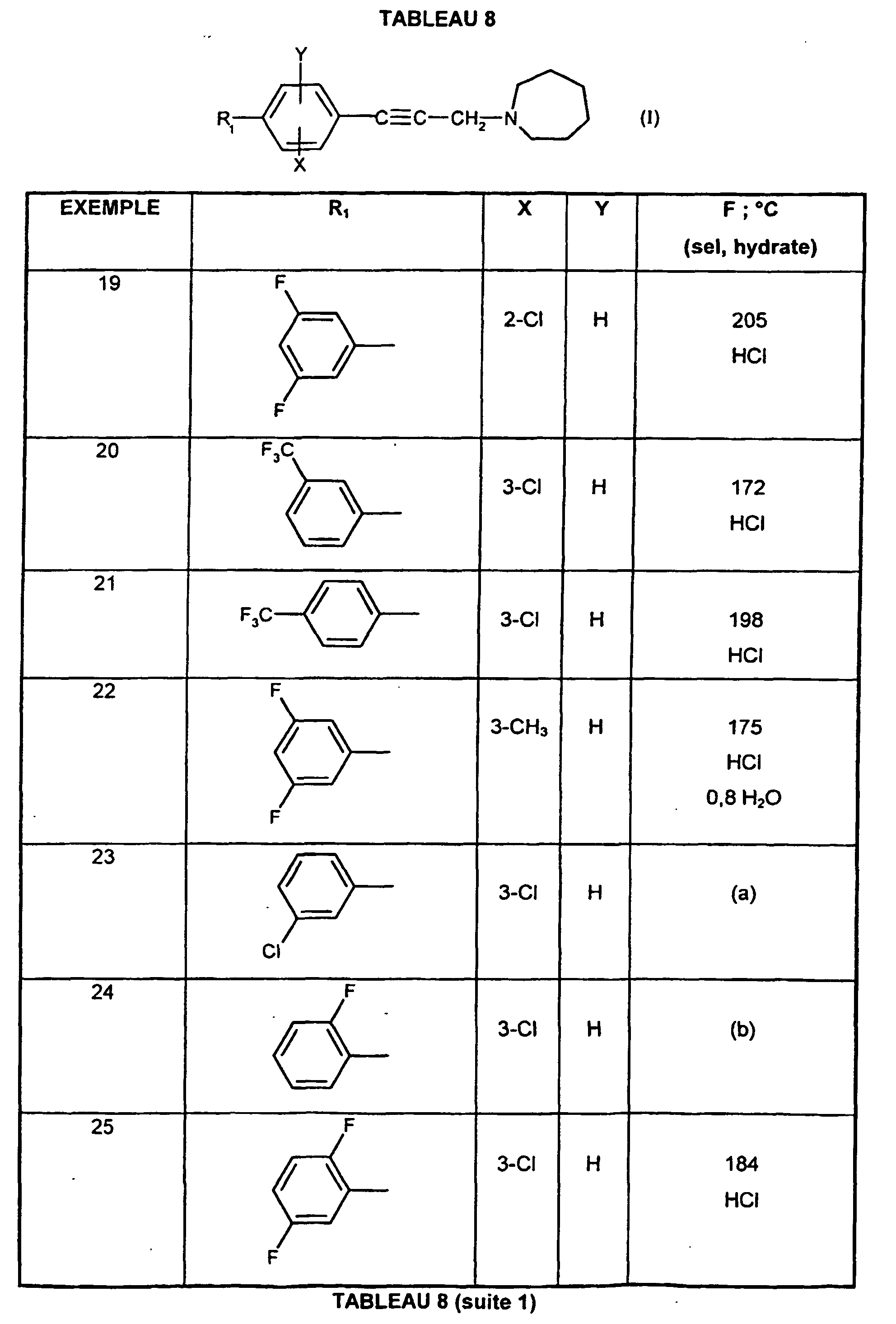

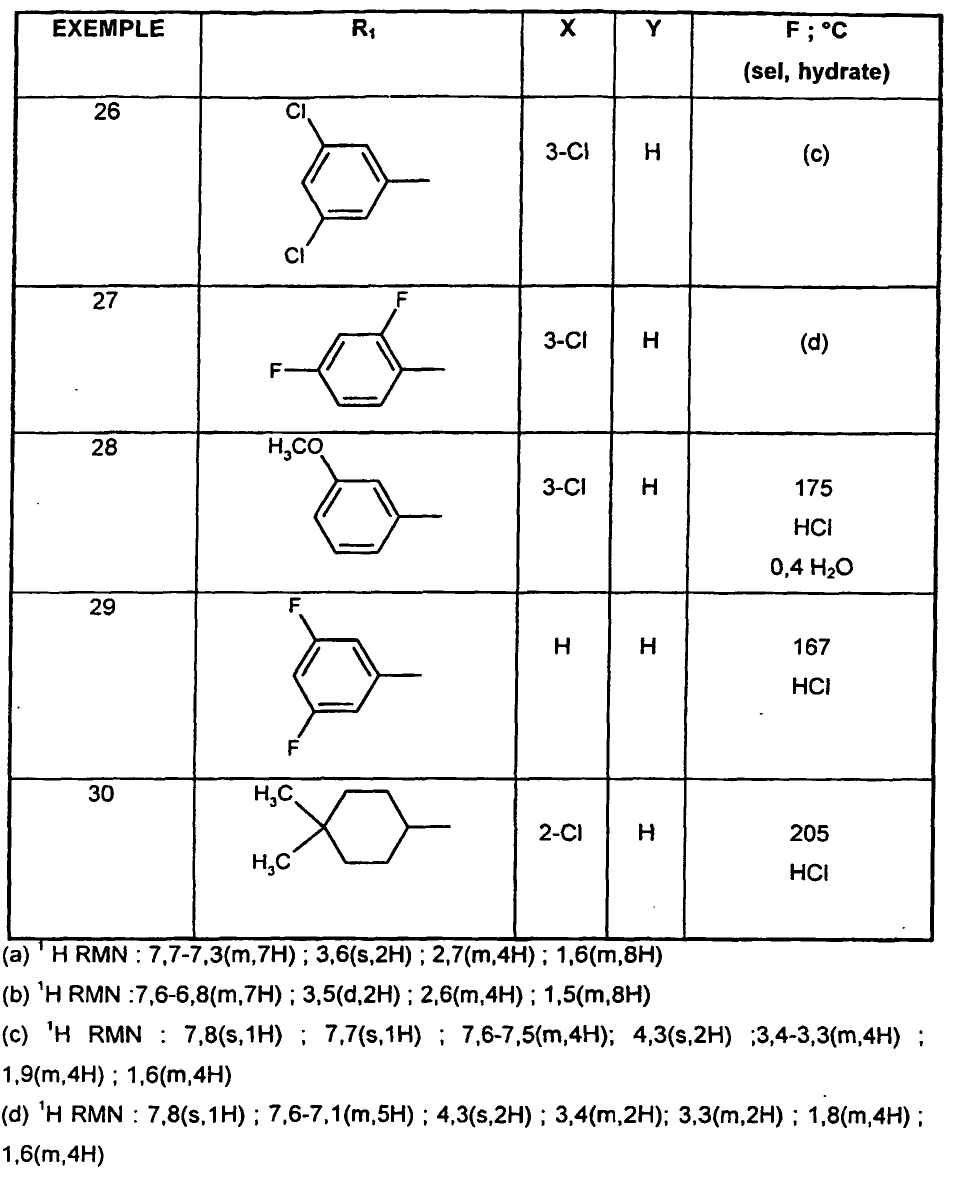

### EXEMPLE 31

### Chlorhydrate de 1-{3-[4-(3,5-difluorophényl)-3-chlorophényl]prop-2-ynyl}azocane

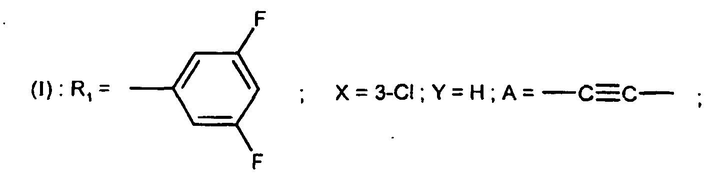

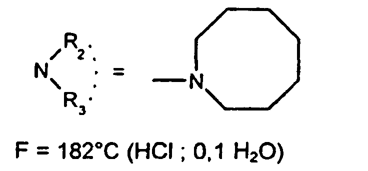

### EXEMPLE 32

### Chlorhydrate de {3-[4-(3,5-difluorophényl)-3-chlorophényl]prop-2-ynyl}pipéridine

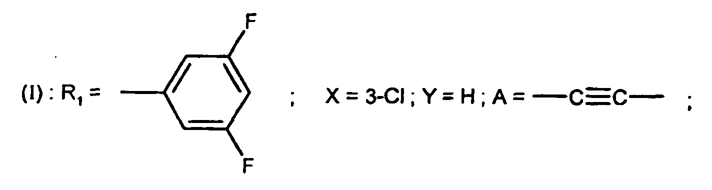

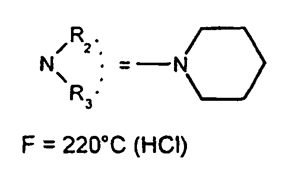

### EXEMPLE 33

### Chlorhydrate de {3-[4-(4,4-diméthylcyclohexyl)-3-chlorophényl]prop-2-ynyl} azépane

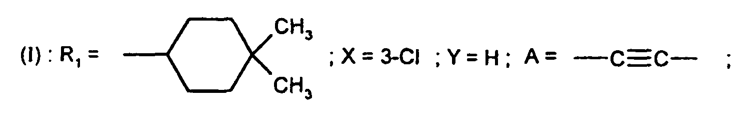

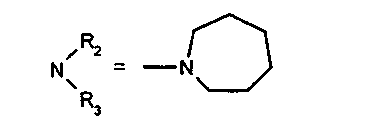

Sous atmosphère inerte, on additionne 0,76 g de dichlorobis(triphénylphosphine) palladium à 3,6 g de 1-prop-2-ynylazépane (*composé 4.1*), 8 g de [4-(4,4-diméthylcyclohexyl)-3-chlorophényl]trifluorométhanesulfonate (*composé III.1*), 0,103 g d'iodure de cuivre, 1,83 g de chlorure de lithium dans 200 ml de triéthylamine et 100 ml de pyridine. Le mélange réactionnel est chauffé à reflux pendant 12 heures. On évapore les solvants sous pression réduite et purifie le résidu obtenu par chromatographie sur une colonne de gel de silice en éluant avec un mélange cyclohexane/acétate d'éthyle 95/5 (v/v). On reprend le résidu obtenu avec de l'éther diéthylique et forme le chlorhydrate en faisant barboter de l'acide chlorhydrique. Après filtration, le résidu obtenu est recristallisé dans le toluène.

### EXEMPLE 34

### Chlorhydrate de 1-{(Z)-3-[3-chloro-4-(3,3,5,5-tétraméthylcyclohexyl)phényl]propèn-2-yl}azépane.

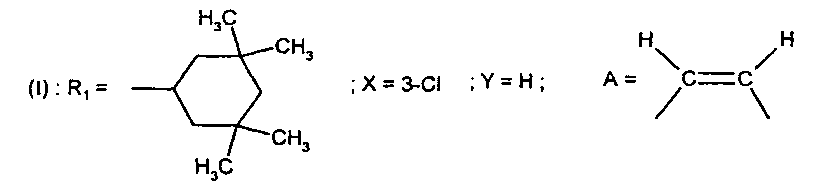

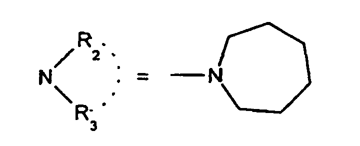

Sous atmosphère inerte et à pression atmosphérique, on hydrogène 2,28 g du composé de l'EXEMPLE 1 dans 40 ml d'éther de pétrole en présence de 2,3 ml de cyclohexène et 0,23 g de palladium sur carbonate de calcium empoisonné par 3,5% de plomb (catalyseur de Lindlar). On filtre sur célite, reprend le résidu huileux obtenu avec du éther diéthylique et fait barboter de l'acide chlorhydrique. On filtre et sèche le précipité sous pression réduite ; F = 190°C (HCl).

De la même manière, on prépare les composés des EXEMPLES 35 à 64 ci-après :

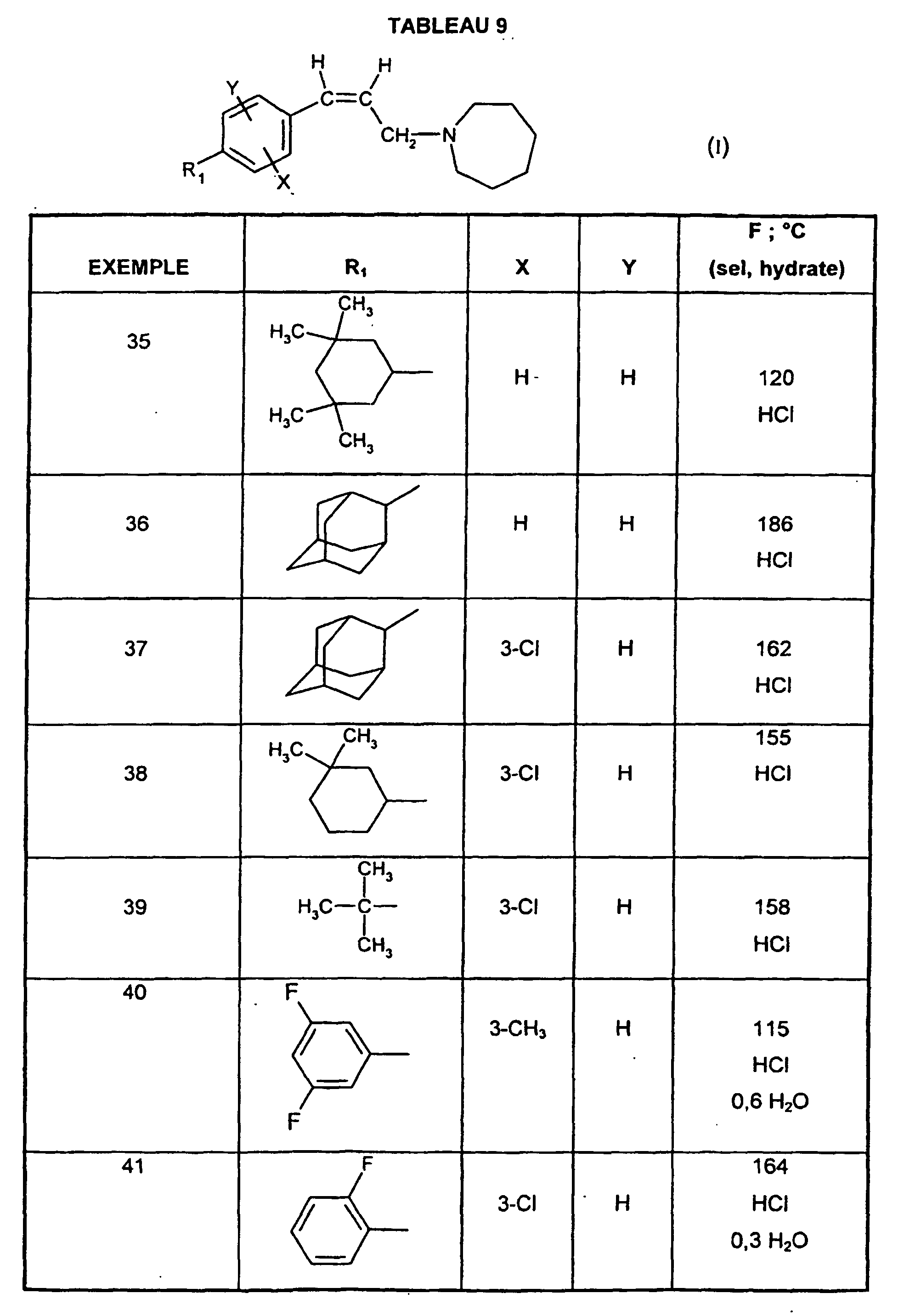

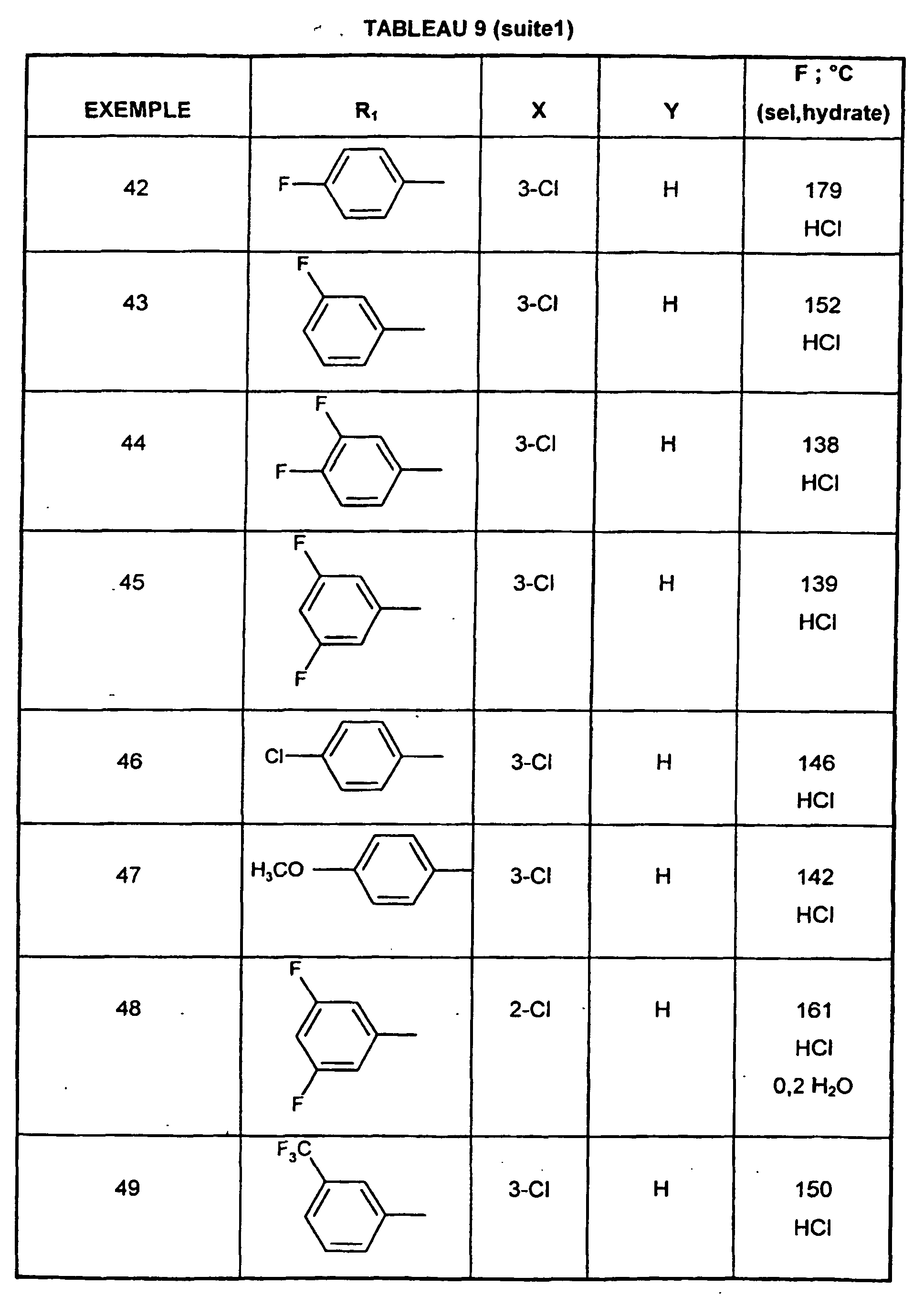

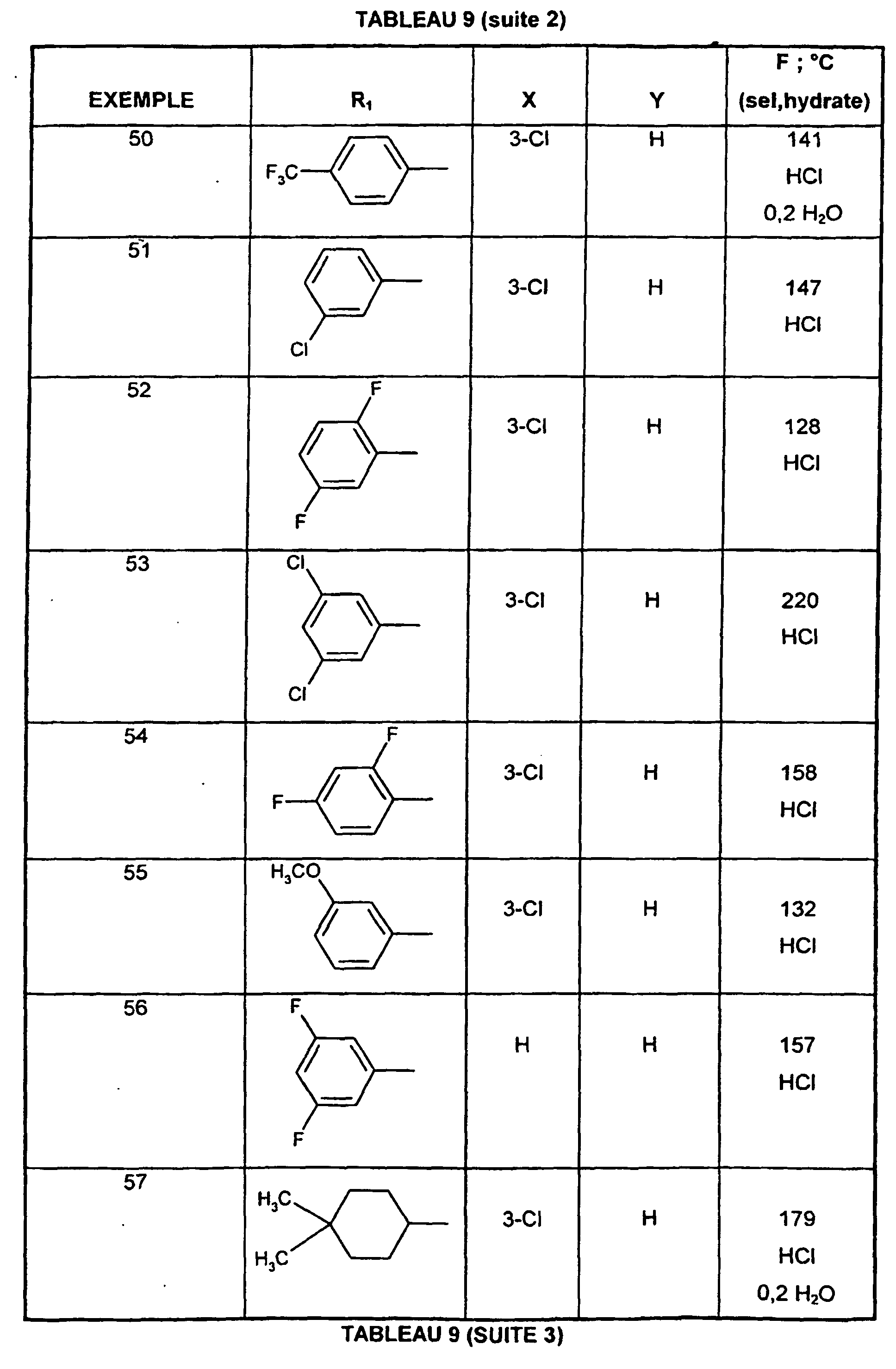

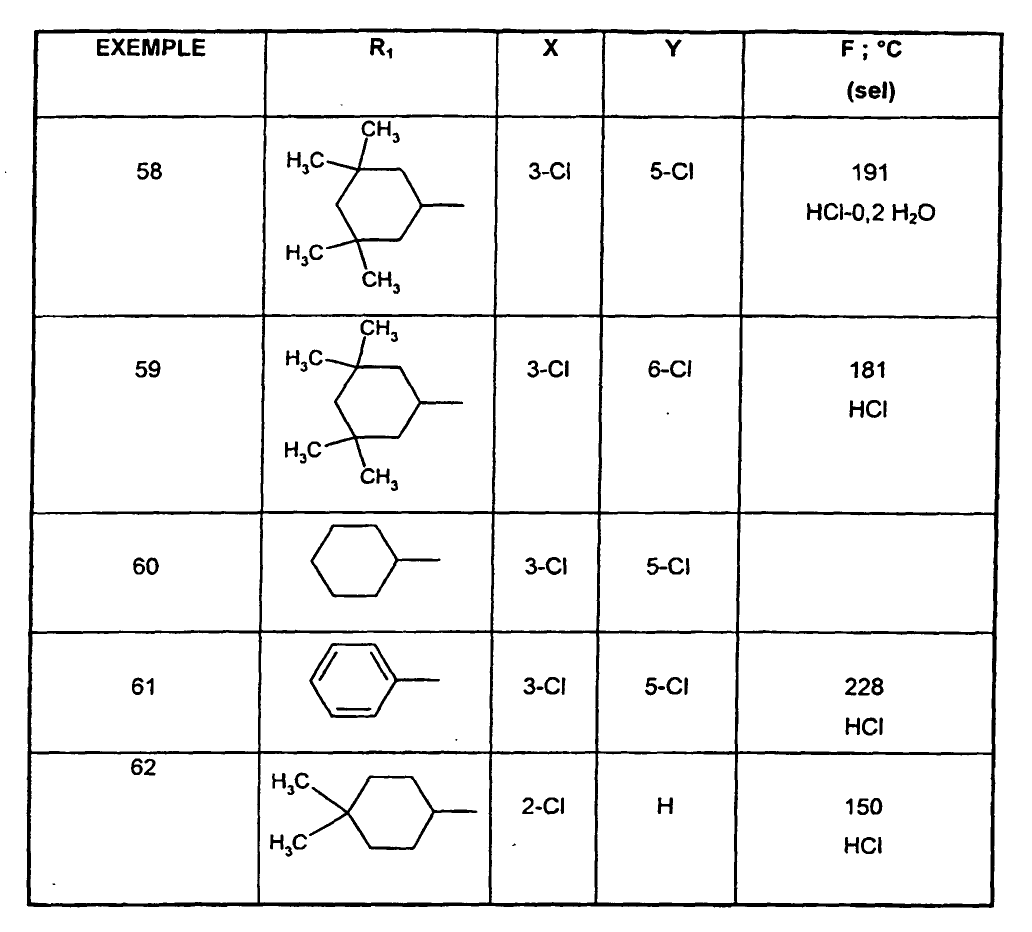

### EXEMPLE 63

### Chlorhydrate de 1-{(Z)-3-[3-chloro-4-(3,5-difluorophényl)phényl]propèn-2-yl}azocane

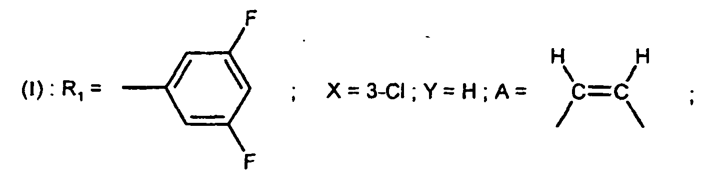

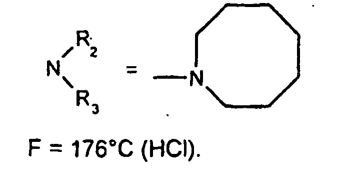

### EXEMPLE 64

### Chlorhydrate de 1-{(Z)-3-[3-chloro-4-(3,5-difluorophényl)phényl]propèn-2-yl} pipéridine

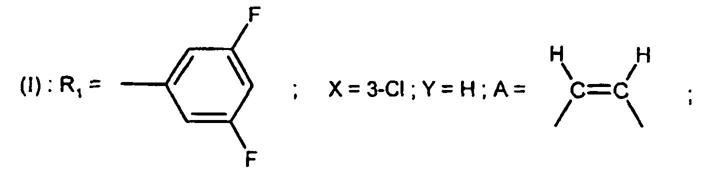

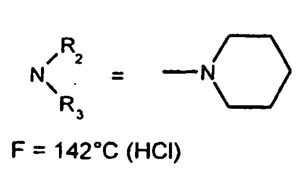

### EXEMPLE 65

### Chlorhydrate de 1-{(E)-3-[3-chloro-4-(3,3,5,5-tétraméthylcyclohexyl)phényl] propèn-2-yl}azépane.

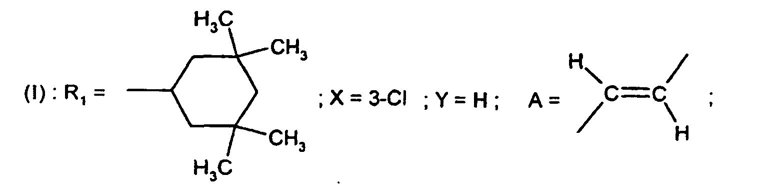

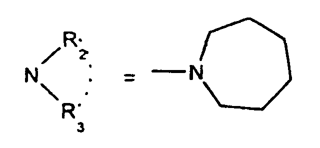

Sous atmosphère inerte, à une solution de 3 g du composé de l'EXEMPLE 1 dans 25 ml de toluène, on additionne goutte à goutte 19,5 ml d'une solution 1 M d'hydrure de diisobutylaluminium (DIBALH) dans le toluène. On agite le mélange réactionnel à 40°C pendant 1 heure puis verse le mélange réactionnel sur un mélange eau/glace. On extrait au dichlorométhane, décante, sèche la phase organique sur sulfate de magnésium et évapore les solvants sous pression réduite. On reprend le résidu avec du éther diéthylique, fait barboter de l'acide chlorhydrique. On filtre et sèche le précipité obtenu ; rdt = 74% ; F = 205°C (HCl).

De la même manière, on prépare les composés des EXEMPLES 66 à 69 présentés dans le TABLEAU 10 ci-après :

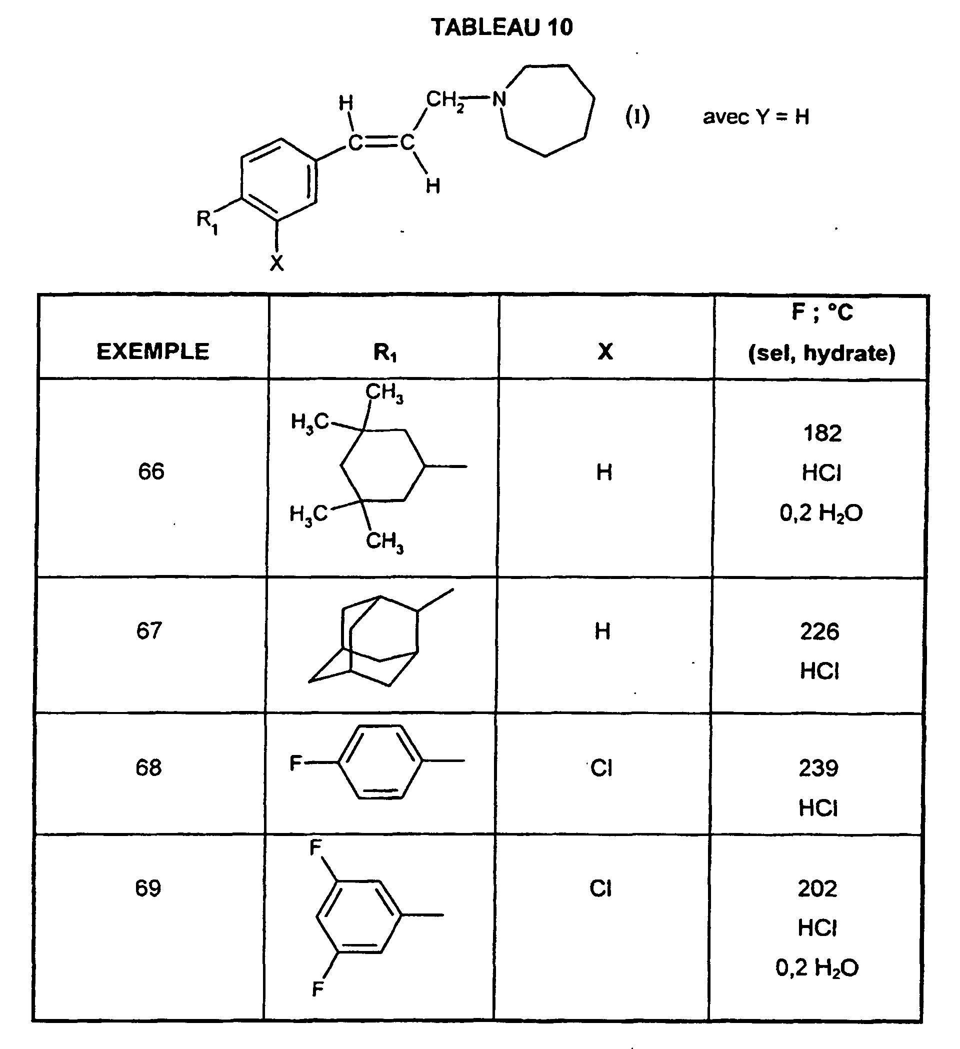

### EXEMPLE 70

### Chlorhydrate de 1-{3-[3-chloro-4-(3,3,5,5-tétraméthyicyclohexyl)phényl]propyl} azépane.

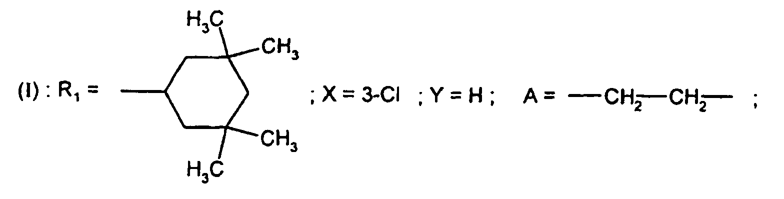

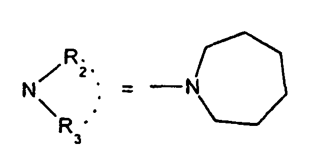

On hydrogène 3,6 g du composé de l'EXEMPLE 1 en présence de 0,36 g de palladium sur charbon à 10% et de 50 ml d'éthanol. On filtre et évapore le filtrat sous pression réduite. On reprend le résidu huileux obtenu avec du éther diéthylique, fait barboter de l'acide chlorhydrique. On filtre et sèche le précipité obtenu ; rdt = 59% ; F = 215°C (HCl).

De la même manière on prépare les composés des EXEMPLES 71 à 85 présentés dans le TABLEAU 11 ci-après :

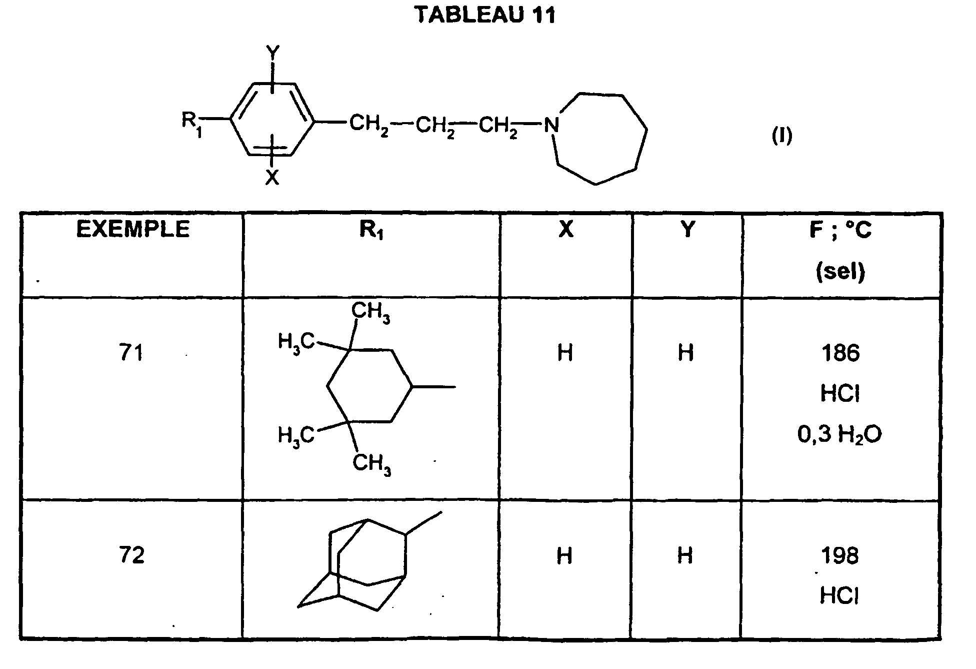

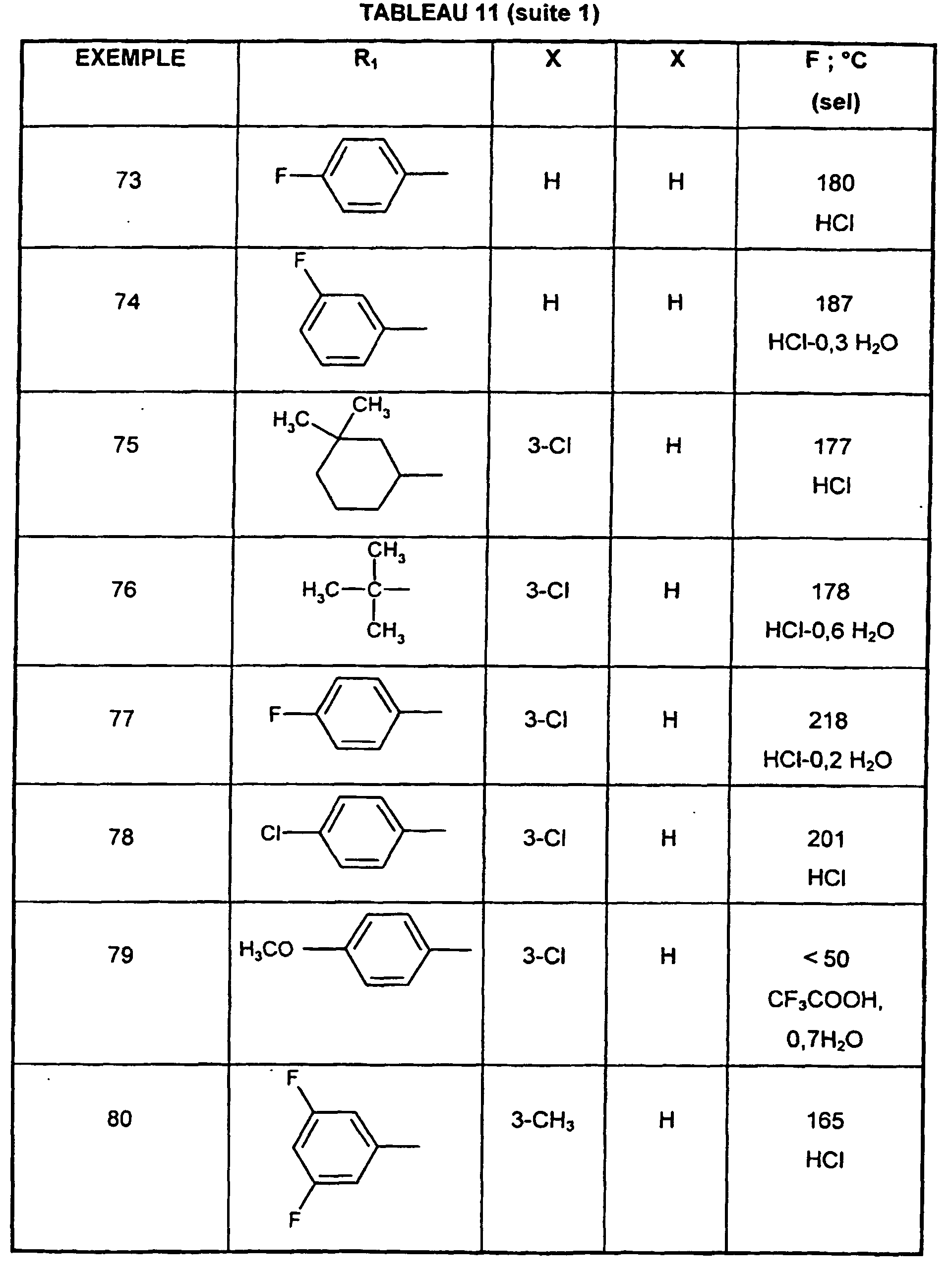

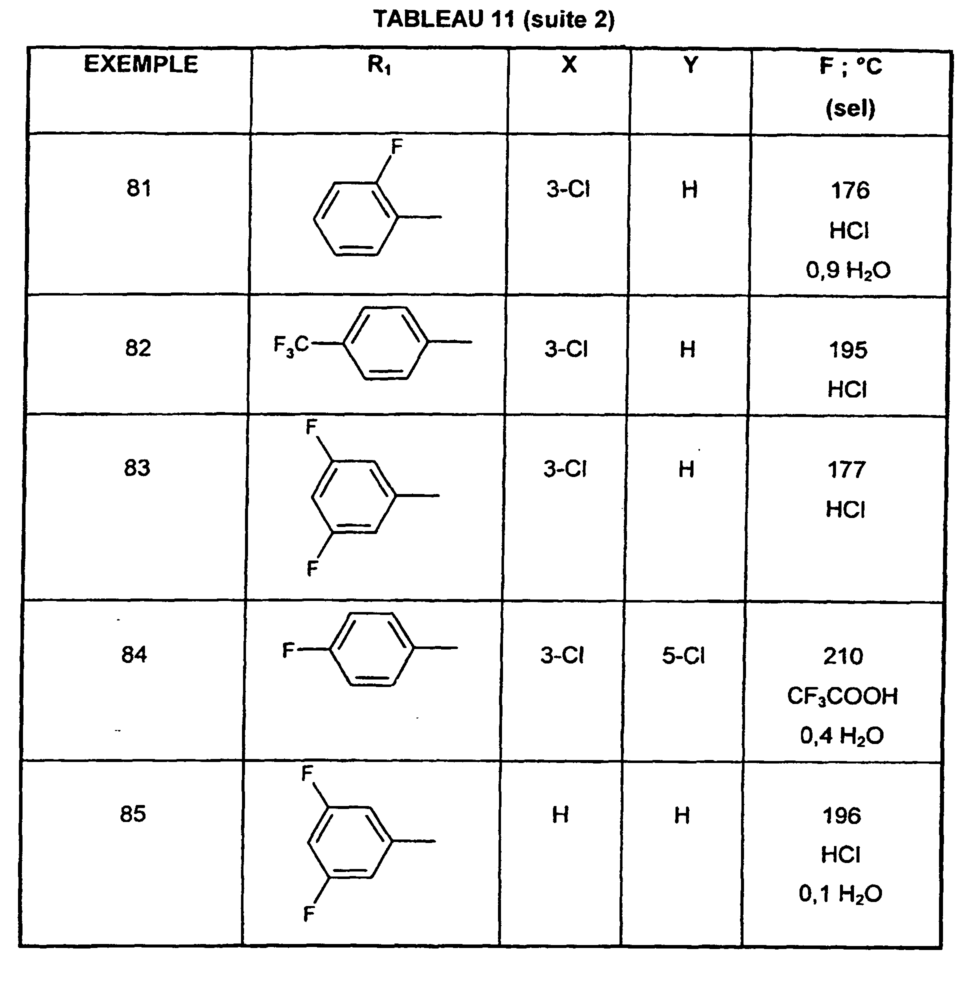

## Revendications

1. Composés de formule : 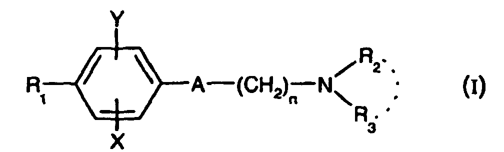 dans laquelle :
- A représente un groupe choisi parmi les suivants :
-C≡C- ; -CH=CH- , -CH₂-CH₂-
- n est égal à 1 ou 2 ;
- X représente un atome d'hydrogène, de chlore ou de fluor ou un groupe méthyle ou méthoxy ;
- Y représente un atome d'hydrogène ou un atome de chlore ou de fluor ;
- R₁ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe (C₁-C₃)alcoxy ou trifluorométhyle ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ; ou R₁ représente un groupe phényle étant entendu que dans ce cas X ou Y est différent de l'hydrogène ; ou bien R₁ représente un groupe cyclohexyle étant entendu que dans ce cas X et Y sont différents de l'hydrogène ;
- R₂ et R₃ forment avec l'atome d'azote auquel ils sont liés une amine cyclique comprenant 5 à 8 chaînons ; un groupe morpholinyle éventuellement substitué en position 3 et 5 par un méthyle ; ou un groupe 4-phényl-1,2,3,6-tétrahydropyridinyle éventuellement substitué sur le phényle par un halogène ou un groupe trifluorométhyle, (C₁-C₄)alkyle ou (C₁-C₄)alcoxy ;
et les sels d'addition de ces composés avec des acides pharmaceutiquement acceptables, ainsi que leurs solvats et hydrates.

2. Composés selon la revendication 1 dans lesquels :
- A représente un groupe choisi parmi les suivants :
-C≡C- ; -CH=CH- ; -CH₂-CH₂-
- n est égal à 1;
- X représente un atome d'hydrogène, de chlore ou un groupe méthyle ;
- Y représente un atome d'hydrogène ou de chlore ;
- R₁ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ou trifluorométhyle ; un groupe *tert*-butyle ou adamantyle 1 ou 2 ; ou R₁ représente un groupe phényle étant entendu que dans ce cas X ou Y sont différents de l'hydrogène ; ou bien R₁ représente un groupe cyclohoxyle étant entendu que dans ce cas X et Y sont différents de l'hydrogène ;
- R₂ et R₃ forment avec l'atome d'azote auquel ils sont liés une amine cyclique comprenant 6 à 8 chaînons ;
et les sels d'addition de ces composés avec des acides pharmaceutiquement acceptables ainsi que leurs solvats et hydrates.

3. Composés selon l'une quelconque des revendications 1 à 2 de formule : 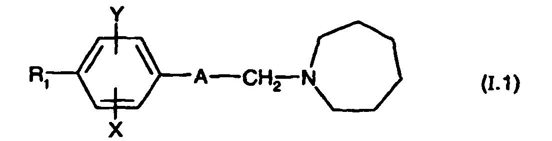 dans laquelle :
- A représente un groupe choisi parmi les suivants :
-C≡C- ; -CH=CH- ; -CH₂-CH₂-
- X représente un atome d'hydrogène ou de chlore ;
- Y représente un atome d'hydrogène ou un atome de chlore ;
- R₁ représente un cyclohexyle monosubstitué, disubstitué, trisubstitué ou tétrasubstitué par un groupe méthyle ; un groupe phényle mono ou disubstitué par un atome de fluor, de chlore ou un groupe méthoxy ; un groupe *tert*-butyle ou adamantyle 1 ou 2 ; R₁ représente un groupe cyclohexyle ou phényle étant entendu que dans ce cas X et Y sont différents de l'hydrogène ;
et les sels d'addition de ces composés avec des acides pharmaceutiquement acceptables, ainsi que leurs solvats et hydrates.

4. Composés selon l'une quelconque des revendications 1 à 3 dans lesquels A représente le groupe -CH=CH- en particulier de configuration (Z).

5. Composés selon l'une quelconque des revendications 1 à 4 dans lesquels X représente un atome de chlore et Y représente un atome d'hydrogène.

6. Composés selon l'une quelconque des revendications 1 à 5 dans lesquels R₁ représente un groupe phényle monosubstitué ou disubstitué par un atome de fluor ou de chlore ou un groupe méthoxy et les sels d'addition de ces composés avec des acides pharmaceutiquement acceptables, ainsi que leurs solvats et hydrates.

7. Composés :
- 1-[(Z)-3-(2-Chloro-3'-fluorobiphényl-4-yl)propèn-2-yl]azépane ;
- 1-[(Z)-3-(2-Chloro-3'-5'-difluorobiphényl-4-yl)propèn-2-yl]azépane ;
- 1-[(Z)-3-(2-Chloro-3'-méthoxybiphényl-4-yl)propèn-2-yl]azépane ; selon la revendication 1, ainsi que leurs sels avec des acides pharmaceutiquement acceptables, solvats et hydrates.

8. Composé 1-[(Z)-3-(2-Chloro-3'-méthoxybiphényl-4-yl)propèn-2-yl]azépane selon la revendication 1, ainsi que ses sels avec des acides pharmaceutiquement acceptables, solvats et hydrates.

9. Composé selon la revendication 8, qui est le fumarate de 1-[(2)-3-(2-chloro-3'-methoxybiphényl-4-yl)-propén-2-yl]azépane.

10. Procédé pour la préparation d'un composé selon la revendication 1 dans lequel A représente le groupe -C≡C- **caractérisé en ce que** :
a) soit, si n = 1, on effectue une réaction de Mannich entre le dérivé phénylacétylénique de formule : 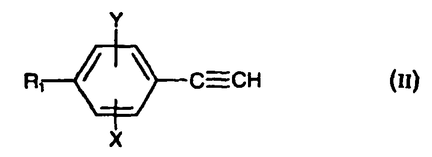 dans laquelle R₁, X et Y sont tels que définis pour (I), le formaldéhyde et l'amine (1) HNR₂R₃, R₂ et R₃ étant tels que définis pour (I) ;
b) soit on effectue un couplage de Suzuki entre le composé de formule : 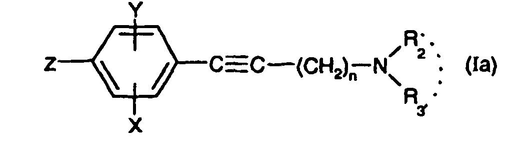 dans laquelle X, Y, n, R₂ et R₃ sont tels que définis pour (I) et Z représente un brome, un iode ou le groupe trifluorométhanesulfonate (OTf) et un dérivé boronique (2) de formule R₁-B(OR)₂ dans lequel R₁ est tel que défini pour (I) et R représente un atome d'hydrogène, un groupe alkyle ou aryle en présence d'une base et d'un catalyseur métallique ;
c) soit lorsque R₁ représente un groupe cyclohexyle monosubstitué, disubstitué, trisubstitué ou tétrasubstitué par un groupe méthyle ; un groupe cycloheptyle, 4-tétrahydropyranyle, 4-tétrahydrothiopyranyle ou adamantyle, on effectue un couplage entre le composé (Ia) dans lequel Z représente un atome d'iode ou de brome avec la cétone (3) correspondant à R₁ représentée par 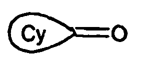 en présence d'une base pour obtenir intermédiairement le composé de formule : 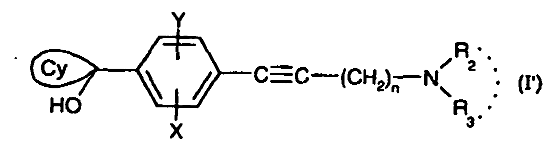 dans laquelle X, Y, n, R₂ et R₃ sont tels que définis pour (I) ; ledit composé (I') étant ensuite réduit dans des conditions sélectives ;
d) soit on effectue une réaction de couplage entre l'amine de formule : 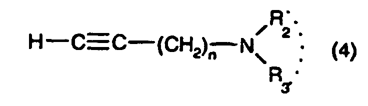
dans laquelle n, R₂ et R₃ sont tels que définis pour (I) et le composé de formule : 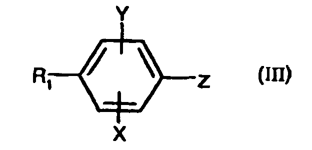 dans laquelle R₁, X et Y sont tels que définis pour (I) et Z représente un atome de brome, iode ou un groupe trifluorométhylsulfonate (triflate ou OTf).

11. Procédé pour la préparation d'un composé selon la revendication 1 dans lequel A représente le groupe -CH=CH-, **caractérisé en ce qu'**on effectue une hydrogénation par l'hydrogène naissant où en présence de cyclohexène du composé (I) dans lequel A représente le groupe acétylénique -C≡C- pour préparer le composé éthylénique (I) sous forme d'un mélange des isomères Z et E ou on effectue cette hydrogénation en présence d'un catalyseur métallique sur support pour préparer le composé éthylénique (I) sous forme Z, ou encore on fait réagir sur le composé (I) dans lequel A représente le groupe acétylénique -C≡C- un hydrure métallique pour préparer le composé éthylénique (I) sous forme E.

12. Procédé pour la préparation d'un composé selon la revendication 1 dans lequel A représente le groupe -CH₂-CH₂-, **caractérisé en ce qu'**on effectue une hydrogénation du composé (I) dans lequel A représente un groupe -CH=CH- ou -C≡C-.

13. Composition pharmaceutique contenant à titre de principe actif un composé selon l'une quelconque des revendications 1 à 9.

14. Utilisation d'un composé selon l'une quelconque des revendications 1 à 9 pour la préparation d'un médicament destiné à traiter les troubles psychiotiques.

15. Utilisation d'un composé selon l'une quelconque des revendications 1 à 9 pour la préparation d'un médicament destiné à traiter les symptômes positifs et négatifs de la schizophrénie.

## Patentansprüche

1. Verbindungen der Formel: 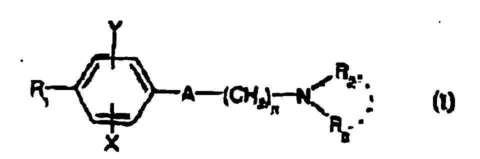 worin:
- A eine Gruppe darstellt, die ausgewählt ist unter den folgenden:
-C≡C-; -CH=CH-; -CH₂-CH₂-
- n gleich 1 oder 2 ist;
- X ein Wasserstoff-, Chlor- oder Fluoratom oder eine Methyl- oder Methoxygruppe darstellt;
- Y ein Wasserstoffatom oder ein Chlor- oder Fluoratom darstellt;
- R₁ eine mit einer Methylgruppe monosubstituierte, disubstituierte, trisubstituierte oder tetrasubstituierte Cyclohexylgruppe; eine mit einem Fluor- oder Chloratom oder mit einer (C₁-C₃)-Alkoxy- oder Trifluormethylgruppe monosubstituierte oder disubstituierte Phenylgruppe; eine Cycloheptyl-, *tert*-Butyl-, Dicyclopropylmethyl-, Bicyclo-[3.2.1]octanyl-, 4-Tetrahydropyranyl-, 4-Tetrahydrothiopyranyl- oder 1- oder 2-Adamantylgruppe darstellt; oder R₁ eine Phenylgruppe darstellt, wobei es sich versteht, dass in diesem Fall X oder Y von Wasserstoff verschieden ist; oder aber R₁ eine Cyclohexylgruppe darstellt, wobei es sich versteht, dass in diesem Fall X und Y von Wasserstoff verschieden sind;
- R₂ und R₃ mit dem Stickstoffatom, an das sie gebunden sind, ein cyclisches Amin mit 5 bis 8 Kettengliedem; eine gegebenenfalls In Position 3 und 5 mit einem Methyl substituierte Morpholinylgruppe; oder eine gegebenenfalls am Phenyl mit einem Halogen oder einer Trifluormethyl-, (C₁-C₄)-Alkyl- oder (C₁-C₄)-Alkoxygruppe substituierte 4-Phenyl-1,2,3,6-tetrahydropyridinylgruppe bilden;
und die Additionssalze dieser Verbindungen mit pharmazeutisch annehmbaren Säuren sowie deren Solvate und Hydrate.

2. Verbindungen gemäß Anspruch 1, worin:
- A eine Gruppe darstellt, die ausgewählt ist unter den folgenden;
-C≡C-; -CH=CH-; -CH₂-CH₂-
- n gleich 1 ist:
- X ein Wasserstoff- oder Chloratom oder eine Methylgruppe darstellt;
- Y ein Wasserstoff- oder Chloratom darstellt;
- R₁ eine mit einer Methylgruppe monosubstituierte, disubstituierte, trisubstituierte oder tetrasubstituierte Cyclohexylgruppe; eine mit einem Fluor- oder Chloratom oder mit einer Methoxy- oder Trifluormethylgruppe monosubstituierte oder disubstituierte Phenylgruppe; eine *tert*-Butyl- oder 1- oder 2-Adamantylgruppe darstellt; oder R₁ eine Phenylgruppe darstellt, wobei es sich versteht, dass in diesem Fall X oder Y von Wasserstoff verschieden sind; oder aber R₁ eine Cyclohexylgruppe darstellt, wobei es sich versteht, dass in diesem Fall X und Y von Wasserstoff verschieden sind;
- R₂ und R₃ mit dem Stickstoffatom, an das sie gebunden sind, ein cyclisches Amin mit 6 bis 8 Kettengliedern bilden;
und die Additionssalze dieser Verbindungen mit pharmazeutisch annehmbaren Säuren sowie deren Solvate und Hydrate.

3. Verbindungen gemäß einem der Ansprüche 1 bis 2 der Formel: 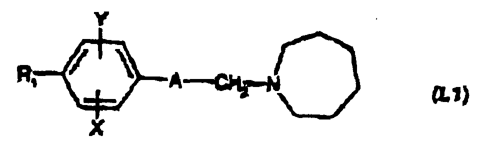 worin:
- A eine Gruppe darstellt, die ausgewählt ist unter den folgenden:
-C≡C-; -CH=CH-; -CH₂-CH₂-
- X ein Wasserstoff- oder Chloratom darstellt;
- Y ein Wasserstoffatom oder ein Chloratom darstellt;
- R₁ eine mit einer Methylgruppe monosubstituierte, disubstituierte, trisubstituierte oder tetrasubstituierte Cyclohexylgruppe; eine mit einem Fluor- oder Chloratom oder einer Methoxygruppe mono- oder disubstituierte Phenylgruppe; eine *tert*-Butyl- oder 1- oder 2-Adamantylgruppe darstellt; R₁ eine Cyclohexyl- oder Phenylgruppe darstellt, wobei es sich versteht, dass in diesem Fall X und Y von Wasserstoff verschieden sind; und die Additionssalze dieser Verbindungen mit pharmazeutisch annehmbaren Säuren sowie deren Solvate und Hydrate.

4. Verbindungen gemäß einem der Ansprüche 1 bis 3, worin A die -CH=CH-Gruppe, Insbesondere mit (Z)-Konfiguration darstellt.

5. Verbindungen gemäß einem der Ansprüche 1 bis 4, worin X ein Chloratom darstellt und Y ein Wasserstoffatom darstellt.

6. Verbindungen gemäß einem der Ansprüche 1 bis 5, worin R₁ eine mit einem Fluor oder Chloratom oder einer Methoxygruppe monosubstituierte oder disubstituierte Phenylgruppe darstellt, und die Additionssalze dieser Verbindungen mit pharmazeutisch annehmbaren Säuren sowie deren Solvate und Hydrate.

7. Verbindungen:
- 1-[(Z)-3-(2-Chlor-3'-fluorbiphenyl-4-yl)propen-2-yl]azepan;
- 1-[(Z)-3-(2-Chlor-3',5'-difluorbiphenyl-4-yl)propen-2-yl]azepan;
- 1-[(Z)-3-(2-Chlor-3'-methoxybiphenyl-4-yl)propen-2-yl]azepan; gemäß Anspruch 1 sowie deren Salze mit pharmazeutisch annehmbaren Säuren, Solvate und Hydrate.

8. Verbindung 1-[(Z)-3-(2-Chlor-3'-methoxybiphenyl-4-yl)propen-2-yl]azepan gemäß Anspruch 1 sowie ihre Salze mit pharmazeutisch annehmbaren Säuren, Solvate und Hydrate.

9. Verbindung gemäß Anspruch 8, die das Fumarat von 1-[(Z)-3-(2-Chlor-3'-methoxybiphenyl-4-yl)propen-2-yl]azepan ist

10. Verfahren zur Herstellung einer Verbindung gemäß Anspruch 1, worin A die -C≡C-Gruppe darstellt, **dadurch gekennzeichnet, dass**:
a) man entweder, wenn n = 1, eine Mannich-Reaktion zwischen dem Phenylacetylen-Derivat der Formel: 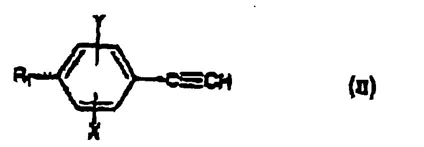 worin R₁, X und Y wie für (I) definiert sind, Formaldehyd und dem Amin (1) HNR₂R₃. wobei R₂ und R₃ wie für (I) definiert sind, ausführt;
b) oder man eine Suzuki-Kopplung zwischen der Verbindung der Formel: 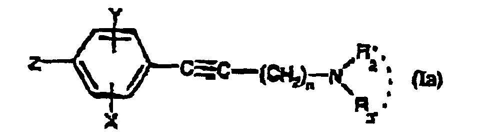 worin X, Y, n, R₂ und R₃ wie für (I) definiert sind und Z ein Brom, lod oder die Trifluormethansulfonatgruppe (OTf) darstellt, und einem Boronderivat (2) der Formel R₁-B(OR)₂, worin R₁ wie für (I) definiert ist und R ein Wasserstoffatom, eine Alkyl- oder Arylgruppe darstellt, in Gegenwart einer Base und eines Metallkatalysators ausführt;
c) oder, wenn R₁ eine mit einer Methylgruppe monosubstituierte, disubstituierte, trisubstituierte oder tetrasubstituierte Cyclohexylgruppe; eine Cycloheptyl-, 4-Tetrahydropyranyl-, 4-Tetrahydrothiopyranyl- oder Adamantylgruppe darstellt, man eine Kopplung zwischen der Verbindung (Ia), worin Z ein lod- oder Bromatom darstellt, mit dem Keton (3), das R₁ entspricht, dargestellt von 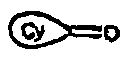 in Gegenwart einer Base ausführt, um intermediär die Verbindung der Formel: 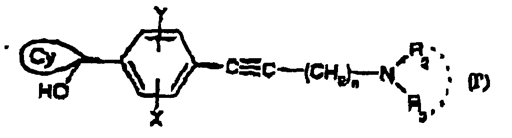 worin X, Y, n, R₂ und R₃ wie für (I) definiert sind, zu erhalten; wobei besagte Verbindung (I') anschließend unter selektiven Bedingungen reduziert wird;
d) oder man eine Kopplungsreaktion zwischen dem Amin der Formel: 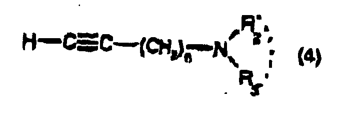
worin n, R₂ und R₃ wie für (I) definiert sind, und der Verbindung der Formel: 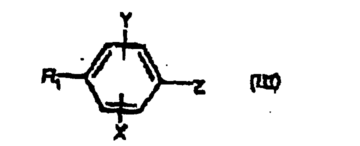 worin R₁, X und Y wie für (I) definiert sind und Z ein Brom- oder lodatom oder eine Trifluormethylsulfonatgruppe (Triflat oder OTf) darstellt, ausführt.

11. Verfahren zur Herstellung einer Verbindung gemäß Anspruch 1, worin A die -CH=CH-Gruppe darstellt, **dadurch gekennzeichnet, dass** man eine Hydrierung mit naszierendem Wasserstoff oder in Gegenwart von Cyclohexen der Verbindung (I), worin A die Acetylengruppe -C≡C- darstellt, ausführt, um die Ethylenverbindung (I) in Form eines Gemischs des Z- und E-Isumers herzustellen, oder man diese Hydrierung in Gegenwart eines trägergebundenen Metallkatalysators ausführt, um die Ethylenverbindung (I) in Z-Form herzustellen, oder man auch die Verbindung (I), worin A die Acetylengruppe -C≡C- darstellt, mit einem Metallhydrid umsetzt, um die Ethylenverbindung (I) in E-Form herzustellen.

12. Verfahren zur Herstellung einer Verbindung gemäß Anspruch 1, worin A die -CH₂-CH₂-Gruppe darstellt, **dadurch gekennzeichnet, dass** man eine Hydrierung der Verbindung (I), worin A eine -CH=CH- oder -C≡C-Gruppe darstellt, ausführt.

13. Pharmazeutische Zusammensetzung, die als Wirkstoff eine Verbindung gemäß einem der Ansprüche 1 bis 9 enthält.

14. Verwendung einer Verbindung gemäß einem der Ansprüche 1 bis 9 für die Herstellung eines Medikaments, das zur Behandlung psychotischer Störungen bestimmt ist.

15. Verwendung einer Verbindung gemäß einem der Ansprüche 1 bis 9 für die Herstellung eines Medikaments, das zur Behandlung der positiven und negativen Symptome der Schizophrenie bestimmt ist.

## Claims

1. Compounds of formula: 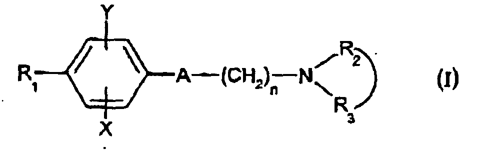 in which:
- A represents a group chosen from the following:
-C≡C- ; -CH=CH- , -CH₂-CH₂-
- n is equal to 1 or 2;
- X represents a hydrogen, chlorine or fluorine atom or a methyl or methoxy group;
- Y represents a hydrogen atom or a chlorine or fluorine atom;
- R₁ represents a cyclohexyl group monosubstituted, disubstituted, trisubstituted or tetrasubstituted with a methyl group; a phenyl group monosubstituted or disubstituted with a fluorine or chlorine atom or with a (C₁-C₃)alkoxy or trifluoromethyl group; a cycloheptyl, *tert*-butyl, dicyclopropylmethyl, bicyclo[3.2.1]octanyl, 4-tetrahydropyranyl, 4-tetrahydrothiopyranyl or 1- or 2-adamantyl group; or R₁ represents a phenyl group, it being understood that, in this case, X or Y is other than hydrogen; or R₁ represents a cyclohexyl group, it being understood that, in this case, X and Y are other than hydrogen;
- R₂ and R₃ form, with the nitrogen atom to which they are bonded, a 5- to 8-membered amine ring; a morpholinyl group optionally substituted in positions 3 and 5 with a methyl; or a 4-phenyl-1,2,3,6-tetrahydropyridyl group optionally substituted on the phenyl with a halogen or a trifluoromethyl, (C₁-C₄)alkyl or (C₁-C₄)alkoxy group;
and the addition salts of these compounds with pharmaceutically acceptable acids, as well as the solvates and hydrates thereof.

2. Compounds according to Claim 1 in which:
- A represents a group chosen from the following:
-C≡C- ; -CH=CH- ; -CH₂-CH₂-
- n is equal to 1;
- X represents a hydrogen or chlorine atom or a methyl group;
- Y represents a hydrogen or chlorine atom;
- R₁ represents a cyclohexyl group monosubstituted, disubstituted, trisubstituted or tetrasubstituted with a methyl group; a phenyl group monosubstituted or disubstituted with a fluorine or chlorine atom or with a methoxy or trifluoromethyl group; a *tert*-butyl or 1- or 2-adamantyl group; or R₁ represents a phenyl group, it being understood that, in this case, X and Y are other than hydrogen; or else R₁ represents a cyclohexyl group, it being understood that, in this case, X and Y are other than hydrogen;
- R₂ and R₃ form, with the nitrogen atom to which they are bonded, a 6- to 8-membered amine ring;
and the addition salts of these compounds with pharmaceutically acceptable acids, as well as the solvates and hydrates thereof.

3. Compounds according to either of Claims 1 and 2 of formula: 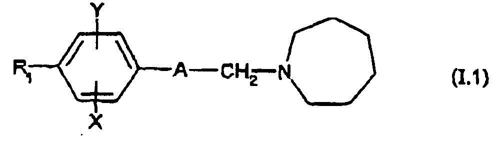 In which:
- A represents a group chosen from the following:
-C≡C- ; -CH=CH- ; -CH₂-CH₂-
- X represents a hydrogen or chlorine atom;
- Y represents a hydrogen atom or a chlorine atom;
- R₁ represents a cyclohexyl group, monosubstituted, disubstituted, trisubstituted or tetrasubstituted with a methyl group; a phenyl group mono- or disubstituted with a fluorine or chlorine atom or a methoxy group; a *tert*-butyl or 1- or 2-adamantyl group; R₁ represents a cyclohexyl or phenyl group, it being understood that, in this case, X and Y are other than hydrogen;
and the addition salts of these compounds with pharmaceutically acceptable acids, as well as the solvates and hydrates thereof.

4. Compounds according to any one of Claims 1 to 3 in which A represents the -CH=CH-group, in particular of (Z) configuration.

5. Compounds according to any of one of Claims 1 to 4 in which X represents a chlorine atom and Y represents a hydrogen atom.

6. Compounds according to any one of Claims 1 to 5 in which R₁ represents a phenyl group monosubstituted or disubstituted with a fluorine or chlorine atom or a methoxy group, and the addition salts of these compounds with pharmaceutically acceptable acids, as well as the solvates and hydrates thereof.

7. Compounds:
- 1-[(Z)-3-(2-chloro-3'-fluorobiphenyl-4-yl)propen-2-yl]azepane;
- 1-[(Z)-3-(2-chloro-3'-5'-difluorobiphenyl-4-yl)propen-2-yl]azepane;
- 1-[(Z)-3-(2-chloro-3'-methoxybiphenyl-4-yl)propen-2-yl]azepane;
according to Claim 1, as well as the salts with pharmaceutically acceptable acids, solvates and hydrates thereof.

8. Compound 1-[(Z)-3-(2-chloro-3'-methoxybiphenyl-4-yl)propen-2-yl]azepane according to Claim 1, as well as the salts with pharmaceutically acceptable acids, solvates and hydrates thereof.

9. Compound according to Claim 8, which is 1-[(Z)-3-(2-chloro-3'-methoxybiphenyl-4-yl)propen-2-yl]azepane fumarate.

10. Method for preparing a compound according to Claim 1 in which A represents a -C≡C-group, **characterized in that**:
a) either, if n = 1, a Mannich reaction is carried out between the phenylacetylene derivative of formula: 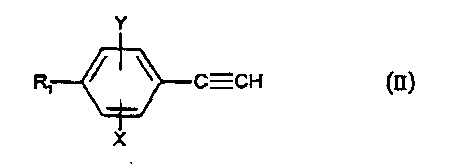 in which R₁, X and Y are as defined for (I), the formaldehyde and the amine (1) HNR₂R₃, R₂ and R₃ being as defined for (I);
b) or a Suzuki coupling is carried out between the compound of formula: 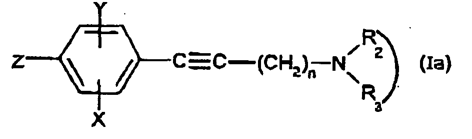 in which X, Y, n, R₂ and R₃ are as defined for (I) and Z represents a bromine, an iodine or a trifluoromethanesulphonate (OTf) group, and a boron derivative (2) of formula R₁-B(OR)₂ in which R₁ is as defined for (I) and R represents a hydrogen atom, an alkyl or aryl group in the presence of a base and a metal catalyst;
c) or, when R₁ represents a cyclohexyl group monosubstituted, disubstituted, trisubstituted or tetrasubstituted with a methyl group; a cycloheptyl, 4-tetrahydropyranyl, 4-tetrahydrothiopyranyl or adamantyl group, a coupling is carried out between the compound (Ia) in which Z represents an iodine or bromine atom and the Ketone (3) corresponding to R₁ represented by 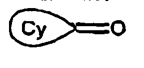 in the presence of a base, to give the intermediate compound of formula: 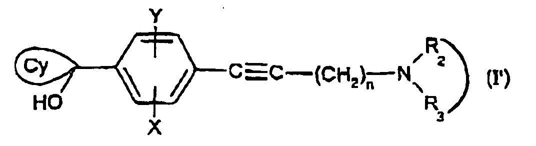 in which X, Y, n, R₂ and R₃ are as defined for (I); said compound (r) then being reduced under selective conditions;
d) or a coupling reaction is carried out between the amine of formula: 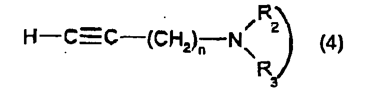
in which n, R₂ and R₃ are as defined for (I), and the compound of formula: 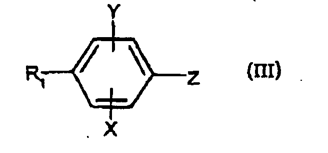 in which R₁, X and Y are as defined for (I) and Z represents a bromine or iodine atom or a trifluoromethylsulphonate (triflate or OTf) group.

11. Method for preparing a compound according to Claim 1 in which A represents a -CH=CH- group, **characterized in that** a hydrogenation with nascent hydrogen or in the presence of cyclohexene is carried out on compound (I) in which A represents an acetylene group -C≡C-, in order to prepare the ethylenic compound (I) in the form of a mixture of the Z and E isomers, or this hydrogenation is carried out in the presence of a metal catalyst on a support in order to prepare the ethylenic compound (I) in Z form, or alternatively compound (I) in which A represents an acetylene group -C≡C- is reacted with a metal hydride in order to prepare the ethylenic compound (I) in E form.

12. Method for preparing a compound according to Claim 1 in which A represents a -CH₂-CH₂- group, **characterized in that** a hydrogenation is carried out on compound (I) in which A represents a -CH=CH- or -C≡C- group.

13. Pharmaceutical composition containing as active principle a compound according to any one of Claims 1 to 9.

14. Use of a compound according to any one of Claims 1 to 9 for preparing a medicinal product intended for treating psychiotic disorders.

15. Use of a compound according to any one of Claims 1 to 9 for preparing a medicinal product intended for treating the positive and negative symptoms of schizophrenia.
